(19) Europäisches Patentamt
European Patent Office
Office européen des brevets

(11) EP 1 873 525 A1

(12) **EUROPEAN PATENT APPLICATION**

(43) Date of publication:
**02.01.2008 Bulletin 2008/01**

(21) Application number: **07012263.5**

(22) Date of filing: **22.06.2007**

(51) Int Cl.:
***G01N 33/543*** *(2006.01)* ***G01N 33/551*** *(2006.01)*
***B01J 19/00*** *(2006.01)*

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK YU**

(30) Priority: **26.06.2006 JP 2006175076**

(71) Applicant: **FUJIFILM Corporation**
**Minato-ku**
**Tokyo (JP)**

(72) Inventors:
- **Ezoe, Toshihide**
**Minato-ku**
**Tokyo (JP)**
- **Yamada, Takayuki**
**Ashigarakami-gun**
**Kanagawa (JP)**
- **Kuruma, Koji**
**Ashigarakami-gun**
**Kanagawa (JP)**

(74) Representative: **HOFFMANN EITLE**
**Patent- und Rechtsanwälte**
**Arabellastrasse 4**
**81925 München (DE)**

(54) **Screening method and screening apparatus**

(57) A method for screening a specified compound, the method comprising: repeatedly applying an assay/washing process to the same biologically active substance for each of different plural compounds, the assay/washing process comprising supplying a compound to an attached biologically active substance, assaying the amount of binding between the biologically active substance and the compound, and washing the compound supplied to the biologically active substance after the assay; and repeating an activity assay for detecting the activity of the biologically active substance at least twice during the interval from before starting of the first assay/washing process to after completion of the last assay/washing process, wherein a relationship of activity change indicating the activity change of the biologically active substance is determined based on the activity of the biologically active substance obtained by the activity assay, a corrected amount of binding is determined by correcting the amount of binding between the biologically active substance and the compound based on the relationship of activity change, and a compound specific to the biologically active substance is sorted based on the corrected amount of binding.

F I G. 9

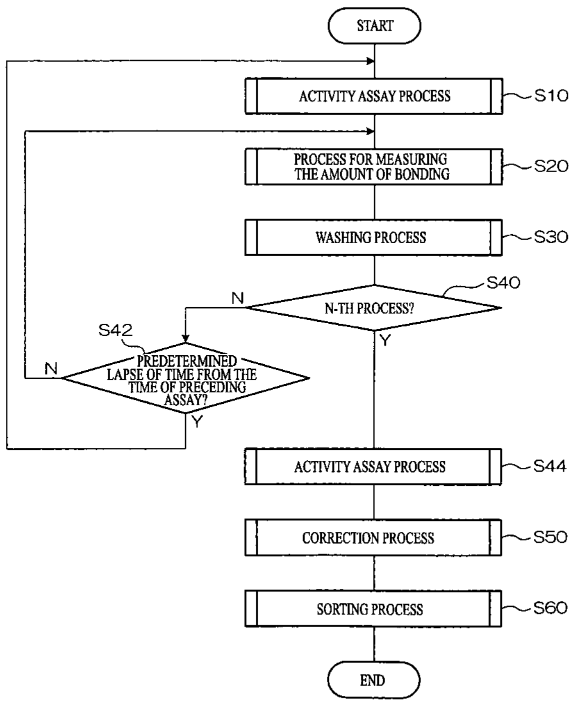


EP 1 873 525 A1

## Description

### BACKGROUND

### Technical Field

The invention relates to a method for sorting a compound that specifically bonded to a biologically active substance from a plurality of compounds, and a screening apparatus.

### Related Art

Various biosensors have been proposed as apparatus for assaying interaction between a given compound and biologically active substances such as proteins. A surface plasmon sensor has been known as one of assay apparatus taking advantage of evanescent wave (Japanese Patent No. 3294605). The surface plasmon sensor usually comprises a prism, a metal film disposed on one surface of the prism on which a biologically active substance is attached, a light source for emitting a light beam, an optical system for permitting the light beam to impinge on the prism so that a total reflection condition is obtained at the interface between the prism and metal film, and a light detecting unit for sensing the intensity of the light beam after the total reflection at the interface. The biologically active substance is assayed based on the detection result of the light detecting unit. A solution of the compound is supplied to the biologically active substance attached on the metal film in the assay using the surface plasmon sensor, the light beam is impinged on a surface of the metal film opposed to the surface on which the solution of the compound is supplied, and interaction between the biologically active substance and the compound in the solution is assayed based on information of the refraction index obtained from the reflected light.

Since the surface plasmon sensor is able to detect fine bonding states between the compounds on the metal film with high precision, the sensor is suitable for large scale screening for sorting a large number of compounds using specific binding ability, if any, of the compounds to a specific biologically active substance after fixing the specified biologically active substance on the metal film.

The same attached biologically active substance may be repeatedly used for assaying different compounds in order to sort a large number of compounds. In this assay method, a compound is supplied for assaying the amount of binding between the biologically active substance and the compound, the preceding compound is removed by washing thereafter, and a succeeding compound is supplied. Compounds exhibiting specific bonding are sorted by repeating the assay/washing process comprising assay and washing.

### SUMMARY

The present invention has been made in view of the above circumstances and provides screening method and screening apparatus.

A first aspect of the present invention provides a method for screening a specified compound, the method comprising:

> repeatedly applying an assay/washing process to the same biologically active substance for each of different plural compounds, the assay/washing process comprising supplying a compound to an attached biologically active substance, assaying the amount of bonding between the biologically active substance and the compound, and washing the compound supplied to the biologically active substance after the assay; and
> repeating an activity assay for detecting the activity of the biologically active substance at least twice during the interval from before starting of the first assay/washing process to after completion of the last assay/washing process, wherein a relationship of activity change indicating the activity change of the biologically active substance is determined based on the activity of the biologically active substance obtained by the activity assay, a corrected amount of binding is determined by correcting the amount of binding between the biologically active substance and the compound based on the relationship of activity change, and a compound specific to the biologically active substance is sorted based on the corrected amount of binding.

A second aspect of the present invention provides a screening apparatus comprising: an assay chip on which a biologically active substance is attached and having formed thereon a liquid pool capable of pooling a liquid on the biologically active substance; an assay/washing unit for repeating an assay/washing process for supplying a compound to the liquid pool and washing the compound supplied to the biologically active substance after assaying the amount of binding between the biologically active substance and the compound for each of different plural compounds with respect to the same biologically active substance; an activity assay unit for detecting the activity of the biologically active substance

at least twice during the interval from before starting of the first assay/washing process to after completion of the last assay/washing process; an activity change relationship calculating unit for determining a relationship of activity change that indicates the activity change of the biologically active substance based on the activity of the biologically active substance detected by the activity assay unit; a bonding amount correction unit for determining a corrected amount of binding by correcting the amount of binding between the biologically active substance and the compound based on the relationship of activity change; and a sorting unit for sorting a compound specific to the biologically active substance based on the corrected amount of binding.

BRIEF DESCRIPTION OF THE DRAWINGS

Exemplary embodiments of the present invention will be described in detail based on the following figures, wherein:

Fig. 1 is a total perspective view of a biosensor pertaining to the exemplary embodiment of the invention;
Fig. 2 is a perspective view of a sensor stick pertaining to the exemplary embodiment of the invention;
Fig. 3 is exploded perspective view of a sensor stick pertaining to the exemplary embodiment of the invention;
Fig. 4 is a cross-sectional view of one liquid flow path portion of the sensor stick pertaining to the exemplary embodiment of the invention;
Fig. 5 shows a light beam impinging on an assay region and reference region of the sensor stick pertaining to the exemplary embodiment of the invention;
Fig. 6 is a perspective view of a pipette portion constituting the liquid supply portion pertaining to the exemplary embodiment of the invention;
Fig. 7 is a schematic illustration of an optical assay portion of the biosensor pertaining to the exemplary embodiment of the invention;
Fig. 8 shows an illustrative block diagram of a control portion and peripheries thereof pertaining to the exemplary embodiment of the invention;
Fig. 9 shows a flow chart of a screening process pertaining to the exemplary embodiment of the invention;
Fig. 10 shows a flow chart of an activity assay process pertaining to the exemplary embodiment of the invention;
Fig. 11 shows a flow chart of a process for assaying the amount of binding pertaining to the exemplary embodiment of the invention;
Fig. 12 shows a flow chart of a washing process pertaining to the exemplary embodiment of the invention;
Fig. 13 shows a flow chart of a correction process pertaining to the exemplary embodiment of the invention;
Fig. 14 is a graph showing a relation between the assay time and activity levels pertaining to the exemplary embodiment of the invention;
Fig. 15 shows a flow chart of a sorting process pertaining to the exemplary embodiment of the invention;
Fig. 16 is a graph showing the relation between the number of assay and activity levels; and
Fig. 17 is a graph showing the relation between the number of assay and activity levels of the protein in an example of the invention.

DETAILED DESCRIPTION

A first aspect of the present invention provides a method for screening a specified compound, the method comprising: repeatedly applying an assay/washing process to the same biologically active substance for each of different plural compounds, the assay/washing process comprising supplying a compound to an attached biologically active substance, assaying the amount of binding between the biologically active substance and the compound, and washing the compound supplied to the biologically active substance after the assay; and repeating an activity assay for detecting the activity of the biologically active substance at least twice during the interval from before starting of the first assay/washing process to after completion of the last assay/washing process, wherein a relationship of activity change indicating the activity change of the biologically active substance is determined based on the activity of the biologically active substance obtained by the activity assay, a corrected amount of binding is determined by correcting the amount of binding between the biologically active substance and the compound based on the relationship of activity change, and a compound specific to the biologically active substance is sorted based on the corrected amount of binding.
The activity assay for detecting the activity of the biologically active substance is repeated at least twice during the interval from before starting of the first assay/washing process to after completion of the last assay/washing process in the screening method of the invention. The activity at the time of the activity assay is detected by this activity assay. However, the activity of the biologically active substance changes with time or at each time that the assay/washing process is repeated. Accordingly, the relationship of activity change indicating the activity change of the biologically active substance is determined based on the detected activity. The relationship of activity change may be based on

time-dependent change of the biologically active substance or change depending on the number of times that the assay/washing process is repeated.

The amount of binding between the biologically active substance and the compound is corrected based on the relationship of activity change. The compound specific to the biologically active substance is sorted based on the corrected amount of binding obtained by the correction.

The compound specifically bonded to the biologically active substance may be more precisely sorted since the invention pays attention to the activity change of the biologically active substance in order to correct the amount of binding between the biologically active substance and the compound by taking the activity change into consideration.

The screening method of the second aspect of the invention, the activity is assayed at least before starting of the first assay/washing process and after completion of the last assay/washing process.

The activity of the biologically active substance is presumed to decrease with time or by repeating the assay/washing process. Accordingly, the highest activity of the biologically active substance may be obtained by assaying the activity before stating of the first assay/washing process. On the other hand, the lowest activity of the biologically active substance may be obtained by assaying the activity after completion of the last assay/washing process. Therefore, the activity of the biologically active substance may be estimated to fall between these two assay points, and the relationship of activity change may be more precisely determined.

The screening methods of to the third and fourth aspects of the invention, the relationship of activity change indicates time-dependent change of the activity, and the corrected amount of binding H is determined by an equation of $H = S/(KT/KN)$, where the activity before starting the assay is represented by a fresh activity KN, the activity at the time of assay is represented by an activity KT, and the amount of binding obtained by the assay is represented by an assayed amount of binding S.

The corrected amount of binding may be determined from the time-dependent activity of the biologically active substance obtained as described above.

The screening apparatus of to the fifth aspect of the invention comprises: an assay chip on which a biologically active substance is attached and having formed thereon a liquid pool capable of pooling a liquid on the biologically active substance; an assay/washing unit for repeating an assay/washing process for supplying a compound to the liquid pool and washing the compound supplied to the biologically active substance after assaying the amount of binding between the biologically active substance and the compound for each of different plural compounds with respect to the same biologically active substance; an activity assay unit for detecting the activity of the biologically active substance at least twice during the interval from before starting of the first assay/washing process to after completion of the last assay/washing process; an activity change relationship calculating unit for determining a relationship of activity change that indicates the activity change of the biologically active substance based on the activity of the biologically active substance detected by the activity assay unit; a bonding amount correction unit for determining a corrected amount of binding by correcting the amount of binding between the biologically active substance and the compound based on the relationship of activity change; and a sorting unit for sorting a compound specific to the biologically active substance based on the corrected amount of binding.

The screening apparatus of the invention comprises the assay chip. The biologically active substance is attached on the assay chip, and the liquid pool capable of pooling the liquid on the biologically active substance is formed on the assay chip. The liquid pool may be a cuvette for pooling the liquid, or may have a liquid flow path for permitting the liquid to flow-in and flow-out.

The assay/washing process in which the compound is supplied to the liquid pool by the assay/washing unit for screening, and in which the compound supplied to the biologically active substance is washed after assay of the amount of binding between the biologically active substance and the compound is repeated for each of different plural compounds with respect to the same biologically active substance.

The activity of the biologically active substance is detected at least twice during the interval from starting of the first assay/washing process to after completion of the last assay/washing process. The relationship of activity change indicating the change of the biologically active substance is determined by the activity change relationship calculating unit based on the detected activity of the biologically active substance. The relationship of activity change may be based on time-dependent change of the biologically active substance or may be based on change depending on the number of tines that the assay/washing processes is repeated.

The corrected amount of binding is determined by correction of the amount of binding between the biologically active substance and the compound by the bonding amount correction unit based on the relation of activity changes, and the compound specific to the biologically active substance is sorted by the sorting unit based on the corrected amount of binding.

Since attention is paid to the activity change of the biologically active substance by the above-mentioned screening apparatus and the amount of binding between the biologically active substance and the compound is corrected by taking the activity change into consideration, the compound specifically bonded to the biologically active substance may be more precisely sorted.

The screening apparatus of to the sixth aspect of the invention further comprises a retrieval unit for retrieving the liquid supplied to the liquid pool.

Providing the retrieval unit permits samples for assaying the activity, which have been subjected to reaction, to be retrieved after allowing the sample for assaying the activity to react with the biologically active substance by supplying the sample to the liquid pool, and the activity may be assayed using the retrieved sample for assaying the activity.

An exemplary embodiment of the present invention will be described below with reference to the drawings.

The biosensor 10 as the screening apparatus pertaining to the exemplary embodiment is a so-called surface plasmon sensor for assaying the interaction between the biologically active substance D and the compound by taking advantage of surface plasmon generated on the surface of a metal film. The biosensor 10 is used for sorting a compound specifically bonded to the biologically active substance D from a large number of compounds A.

As shown in Fig. 1, the biosensor 10 is disposed with a tray holding unit 12, a conveyance unit 14, a container platform 16, a liquid drawing/dispensing unit 20, an optical assay unit 54, and a control unit 60.

The tray holding unit 12 is configured to include a platform 12A and a belt 12B. The platform 12A is attached to the belt 12B, which is disposed in the direction of arrow Y, and is configured to be movable in the direction of arrow Y by the rotation of the belt 12B. Trays T are placed on the platform 12A. Sensor sticks 40 are housed in the trays T. The sensor sticks 40 are chips in which the biologically active substance D are fixed, and the details thereof will be described later. A pushup mechanism 12D that pushes up the sensor sticks 40 as far as the position of a later-described stick holding member 14C is disposed below the platform 12A.

As shown in Fig. 2 and Fig. 3, each of the sensor sticks 40 is configured by a dielectric block 42, a flow path member 44, and a holding member 46.

The dielectric block 42 is configured by transparent resin or the like that is transparent to light beams and is disposed with a prism portion 42A whose cross section is shaped like a trapezoidal rod and held portions 42B that are formed on both end portions of the prism portion 42A integrally with the prism portion 42A. A metal film 57 is formed on the upper surface of the wider of two mutually parallel surfaces of the prism portion 42A. The dielectric block 42 functions as a prism such that, during assay by the biosensor 10, light beams are made incident from one of two side surfaces of the prism portion 42A that face each other but are not mutually parallel, and light beams totally reflected by the boundary with the metal film 57 are emitted from the other side.

As shown in Fig. 4, a linker layer 57A is formed on the surface of the metal film 57. The linker layer 57A is a layer for fixing biologically active substance D onto the metal film 57.

An engagement projection 42C to be engaged with the holding member 46 is formed on each of the side faces of the prism portion 42A along the upper edge thereof. A flange portion 42D to be engaged with a conveyance rail (not shown) is formed on the lower side of the prism portion 42A along the side edge thereof.

As shown in Fig. 3(B), the flow path member 44 is disposed with six base portions 44A, and four circular cylinder members 44B are erectly disposed on each of the base portions 44A. The upper portion of one of the erectly disposed circular cylinder members 44B on each of the base portions 44A is coupled by a coupling member 44D for every three of the base portions 44A. The flow path member 44 is configured by a soft, elastically deformable material such as an amorphous polyolefin elastomer.

As shown in Fig. 5, two generally S-shaped flow path grooves 44C are formed in the bottom surface of each of the base portions 44A. End portions of each of the flow path grooves 44C are communicated with a hollow portion in one of the circular cylinder members 44B. The bottom surface of each of the base portions 44A is tightly adhered to the upper surface of the dielectric block 42, and liquid flow paths 45 are configured by the hollow portions and by spaces configured between the flow path grooves 44C and the upper surface of the dielectric block 42. Two liquid flow paths 45 are configured in one base portion 44A. In each of the liquid flow paths 45, an exit/entry opening 43 of the liquid flow path 45 is configured in the upper end surface of each of the circular cylinder members 44B.

Here, of the two liquid flow paths 45, one is used as an assay flow path 45A and the other is used as a reference flow path 45R. Assay is performed in a state where the biologically active substance D are fixed onto the metal film 57 of the assay flow path 45A and where the biologically active substance D are not fixed onto the metal film 57 of the reference flow path 45R. As shown in Fig. 5, light beams L1 and L2 are made incident respectively at the assay flow path 45A and the reference flow path 45R. As shown in Fig. 6, curved portions of the S shapes disposed on a midline M of the base portion 44A are irradiated by the light beams L1 and L2. Below, the region of the flow path 45A irradiated by the light beam L1 will be called an "assay region E1" and the region of the flow path 45R irradiated by the light beam L2 will be called a "reference region E2". The reference region E2 is a region where assay for correcting data obtained from the assay region E1 where the biologically active substance D are fixed is performed.

The holding member 46 has an elongate shape and includes an upper surface member 47 and two side surface plates 48 that are configured like a lid. Engagement holes 48C that engage with the engagement projections 42C of the dielectric block 42 are formed in the side surface plates 48, and windows 48D are formed in portions of the side surface plates 48 that correspond to the light paths of the light beams L1 and L2. The holding member 46 is attached to the dielectric block 42 as a result of the engagement holes 48C and the engagement projections 42C engaging with each

other. It will be noted that, as described later, the flow path member 44 is molded integrally with the holding member 46 and is disposed between the holding member 46 and the dielectric block 42.

Receiving portions 49 are formed in the upper surface member 47 at positions corresponding to the circular cylinder members 44B of the flow path member 44. As shown in Fig. 4, each of the receiving portions 49 has a substantially circular cylinder-like shape, and the circular cylinder members 44B are disposed in hollow lower portions of the receiving portions 49. Further, a recessed portion 49A that communicates with the exit/entry opening 43 is configured further toward the upper side of each of the receiving portions 49 than the hollow circular cylinder members 44B. Pipette tips 50 are inserted into the recessed portions 49A.

The holding member 46 is configured by a material that is harder than that of the flow path member 44, such as a crystalline polyolefin.

As shown in Fig. 6, each of the pipette tips 50 has a substantially conical cylinder-like shape and is configured by a distal end portion 51, a body portion 52, and a holding portion 53. The distal end portion 51 has a circular cylinder-like shape, and an opening 51 A that dispenses or draws in liquid is configured in the most distal end of the distal end portion 51 in the insertion direction. The body portion 52 has a conical cylinder-like shape whose outer periphery is larger than that of the distal end portion 51, and an outer peripheral step portion 52A is configured between the body portion 52 and the distal end portion 51. The outer peripheral step portion 52A is configured in a tapered shape whose distal end portion 51 side has a small diameter. The outer periphery of the holding portion 53 has a larger diameter than that of the body portion 52, and a holding step portion 53A is configured between the holding portion 53 and the body portion 52. The holding step portion 53A is a portion that is used when holding the pipette tip 50 in a pipette tip stocker including an upper surface plate in which an unillustrated holding hole is configured.

As shown in Fig. 4, the recessed portion 49A of each of the receiving portions 49 is configured so as to be surrounded by a first inner wall portion 49B at the flow path member 44 side and a second inner wall portion 49C. The first inner wall portion 49B has a shape along the distal end portion 51 where its length along an insertion direction Z of the pipette tip 50 is slightly longer than that of the distal end portion 51 of the pipette tip 50 and has a slightly larger diameter than the outer diameter of the distal end portion 51.

The second inner wall portion 49C is configured by an inner peripheral step portion 49D between the first inner wall portion 49B and the second inner wall portion 49C, a central inner wall portion 49E adjacent to the inner peripheral step portion 49D, and an uppermost upper wall portion 49F. The inner peripheral step portion 49D is continuous from the first inner wall portion 49B and is configured in a tapered shape whose upper portion has a large diameter along the outer peripheral step portion 52A of the pipette tip 50. The central inner wall portion 49E is continuous with the inner peripheral step portion 49D and is configured in a tapered shape whose portion above the pipette tip 50 has a large diameter. The upper inner wall portion 49F is continuous with the central inner wall portion 49E and is configured in a tapered shape whose portion above the pipette tip 50 has an even larger diameter.

The holding member 46 and the flow path member 44 are integrally molded by two-color molding (double molding) where different materials are combined together and molded inside the same mold.

As shown in Fig. 1, the conveyance unit 14 of the biosensor 10 is configured to include an upper guide rail 14A, a lower guide rail 14B, and a stick holding member 14C. The upper guide rail 14A and the lower guide rail 14B are disposed above the tray holding unit 12 and the optical assay unit 54 and horizontally in the direction of arrow X, which is orthogonal to the direction of arrow Y. The stick holding member 14C is attached to the upper guide rail 14A. The stick holding member 14C is configured such that it is capable of holding the held portions 42B on both end portions of each of the sensor sticks 40 and is movable along the upper guide rail 14A. The lower guide rail 14B engages with the flange portion 42D in the sensor stick 40 held in the stick holding member 14C, and the stick holding member 14C moves in the direction of arrow X, whereby the sensor stick 40 is conveyed to an assay unit 56 on the optical assay unit 54. Further, the assay unit 56 is disposed with a holding member 58 that holds the sensor stick 40 during assay. The holding member 58 is configured to be movable in the Z direction by an unillustrated drive mechanism and pushes from above the sensor stick 40 disposed in the assay unit 56.

An analyte solution plate 17, a buffer liquid stocker container 18, and a waste liquid container 19 are placed on the container platform 16. The analyte solution plate 17 is partitioned into a matrix and stocked with various kinds of analyte solutions. The buffer liquid stocker container 18 is configured by plural containers and stocked with plural kinds of buffer liquids having different refractive indexes. Openings H into which the later-described pipette tips 50 are insertable are formed in the buffer liquid stocker container 18. The waste liquid container 19 is configured by plural containers and, similar to the buffer liquid stocker container, openings H into which the pipette tips 50 are insertable are formed therein.

The liquid drawing/dispensing unit 20 comprises a head 24 as shown in Fig. 1. The head 24 is movable in the direction of arrow Y (see Fig. 1) along the conveyance rail 22. The head 24 is also movable in the vertical direction (in the direction of arrow Z) by means of a driving mechanism (not shown) within the head 24.

The liquid is supplied to the liquid flow path 45 in the head 24 by inserting two pipette tips 50 into two respective openings of the liquid flow path 45, and the liquid is dispensed from one of pipette tips 50 while the liquid in the liquid flow path 45 is drawn with the other pipette tip 50.

While the liquid is supplied to the sensor stick 40 using the pipette tip 50 in this exemplary embodiment, an injection tube one end of which is connected to each liquid plate and the other end of which is connectable to the sensor stick 40 may be provided in place of the pipette tip in order to supply the liquid with a pump.

The optical assay unit 54 is configured so as to contain a light source 54A, a first optical system 54B, a second optical system 54C, a light receiving unit 54D and a signal processing unit 54E. A divergent light beam L is emitted from the light source 54A. The light beam L is split into two light beams L1 and L2 through the first optical system 54B, and the split beams impinge on the assay region E1 and reference region E2 of the dielectric block 42 disposed on the assay portion 56, respectively. The light beams L1 and L2 on the assay region E1 and reference region E2 contain various incident angle components against the interface between the metal film 57 and dielectric block 42 while the beams impinge at angles larger than a total reflection angle. Therefore, light beams L1 and L2 are totally reflected at the interface between the dielectric block 42 and metal film 57. Totally reflected light beams L1 and L2 are also reflected with various reflection angle components. Totally reflected light beams L1 and L2 are received with the light receiving unit 54D through the second optical system 54C and the optical energy of each light beam is converted into a photoelectric detection signal, which is sent to a signal processing unit 54E. The signal processing unit 54E processes the signal in a predetermined manner based on the received photoelectric detection signals, and assay data G 1 of the assay region E1 and reference data G2 of the reference region E2 are determined. The assay data G1 and reference data G2 are sent to a control unit 60.

The control unit 60 has a function for controlling the entire biosensor 10, and is connected to the light source 54A, signal processing unit 54E and a driving system (not shown) of the biosensor 10 as shown in Fig. 7. The control unit 60 comprises a CPU 60A, a ROM 60B, a RAM 60C, a memory 60D and an interface I/F 60E connected to one another through a bas B as shown in Fig. 8, and is connected to a display unit 62 which displays various information and to an input unit 64 for receiving information.

The memory 60D stores various programs for controlling the biosensor 10 and various data.

An activity assay unit 63 is connected to the control unit 60. The activity assay unit 63 is provided for the assay of an activity K of the biologically active substance D. A container 13 for retrieving an activity assay sample used is disposed on the optical assay unit 54, whereby the activity is assayed by injecting the activity assay sample retrieved from the liquid flow path 45. The activity K of the biologically active substance D is assayed by measuring the fluorescence intensity of the activity assay sample retrieved into the container 13 for retrieving the activity assay sample.

The activity K decreases with time or by repeating the assay/washing steps. Fig. 16 shows data demonstrating that the amount of binding of an inhibitor (SB 203580) against p38 MAP kinase decreases by repeating the assay/ washing steps. The data show that the activity decreases to 81.5% at 44-th step relative to an activity K of 100 at the first step by repeating the first to 44-th assay/washing steps. Accordingly, the biologically active substance D is screened by taking the decrease of the activity K into consideration in this exemplary embodiment.

Screening using the biosensor 10 pertaining to this exemplary embodiment will be described below.

The same biologically active substance D is attached on each of the liquid flow path 45 of one sensor stick 40 in this exemplary embodiment. The amounts of bonding are assayed by supplying solutions of different compounds YA to respective liquid flow paths 45 disposed on the sensor stick 40, compound A is removed from the biologically active substance D by a washing step thereafter, and a solution YA of a succeeding compound is supplied for the assay of the amount of binding of the next compound followed by washing the compound. This assay/washing process is repeated plural times (N-times).

The activity of the biologically active substance D is assayed at least twice during the interval from before starting of the first assay/washing process to after completion of the last assay/washing process. The activity is assayed before starting of the first assay/washing process, each time that a predetermined amount of time elapses after starting of the first assay/washing process, and after completion of the last assay/washing process in this exemplary embodiment.

The screening process shown in Fig. 9 is implemented at the control unit 60 when the sensor stick 40 is conveyed to the assay unit 56 and instruction for starting of the assay is sent from the input unit 64.

The activity is assayed at step S10 at first in the screening process. Examples of the assay method of the activity include a method for assaying an enzyme activity and a method for assaying a receptor activity.

The principles of the enzyme activity assay are described in "Tanpakushitsu, Koso no Kiso Jikkenhou (Basic Experimental Methods of Proteins and Enzymes)", Chapter IV, by Takeichi Horio and Jinpei Yamashita. The detection methods include (1) a spectroscopic assay method, (2) a fluorescence method, (3) an electrode method and (4) a chemoluminescence method, and the methods described, for example, in "Shin Seikagaku Jikken Koza, Tanpakushitsu V (New Guide Book for Biochemical Experiments, Protein V)", "Enzyme Immunoassay Method, Third Edition", and "Enzaimu Imunoassei, Seikagaku Jikkenhou (Enzyme Immunoassay, Methods of Biochemical Experiments)" may also be used.

When the enzyme activity is assayed by the fluorescence method, for example, a substrate specific to the enzyme (a substrate that emits fluorescence from only a decomposition product thereof resulting from decomposition by the enzyme) is allowed to react with the enzyme, and fluorescence of the decomposition product is assayed.

When the enzyme activity is assayed by the spectroscopic method, an initial reaction rate is determined for the assay of the enzyme activity by measuring time-dependent change of absorbance by taking advantage of a difference in spectroscopic properties between the substrate and reaction product.

When the enzyme activity is assayed by the electrode method using an automatic titrator, pH change of a sample solution caused by acids or bases formed as a result of chemical reactions including the enzyme reaction may be electrically detected in order to assay the enzyme reaction.

When the enzyme activity is assayed by the chemoluminescence method, an antibody or an antigen is labeled with the enzyme, a chemoluminescent substrate (a substrate that emits light from only a decomposition product there of resulting from decomposition by the enzyme) specific to the enzyme is allowed to react with the enzyme after the antigen-antibody reaction, and chemoluminescence of the decomposition product is measured in order to assay the enzyme activity.

When the receptor activity is measured, at least one of receptors or ligands attached on a support is made to contact a test sample that contains a ligand or receptor labeled with an antigen, and the enzyme-labeled ligand or receptor, which is attached on the support and bonded to at least one of the receptors or ligands, is made to contact an antibody against the antigen (for example an antibody labeled with an enzyme used for detection) in order to permit the antigen to react with the antibody. The ligand or receptor in the test sample may be detected by detecting the presence of the antibody bonded by the above-mentioned reaction.

From the enzyme activity is assayed by the fluorescence assay of the decomposition product among the above-mentioned assay methods in this exemplary embodiment. As shown in Fig. 10, a signal for supplying the activity assay sample is sent in step S12. The head 24 is actuated by receiving the signal, and the activity assay sample stored in the buffer solution container 18 is supplied to the liquid flow path 45 by means of the pipette tip 50. Then, an activity assay time T during which this process is implemented is stored in step S 13. The activity assay time T is stored as T0, T1, T2 and so on every time that the activity assay process is implemented. The assay is suspended in step S 14 until a predetermined time passes. This suspension time is necessary for decomposing of the substrate in the activity assay sample by the enzyme. After a predetermined lapse of time, a signal for retrieving the activity assay sample is sent in step S 15, and the activity assay sample in the liquid flow path 45 is retrieved. The sample is retrieved by inserting two pipette tips 50 of the head 24 into the liquid flow path 45, injecting a buffer solution from one of the pipette tips 50 while drawing the activity assay sample from the other pipette tip 50, and injecting the drawn activity assay sample into the container 13 for retrieving an activity assay sample. Then, an assay instruction signal of the activity K is sent to the activity assay unit 63 in step S16. Fluorescence of the retrieved activity assay sample is measured at the activity assay unit 63, and the assay result is sent to the control unit 60 as the activity K. The control unit 60 is waiting in step S 17 until it receives the activity K, and the input value is stored in memory 60D in step S 18 in correspondence with the time stored in step S13 after input of the activity.

The activity K of the biologically active substance D at a given time is stored by the above-mentioned activity assay process.

The amount of binding is measured in step S20 in Fig. 9. As shown in Fig. 11, a signal for instructing supply of the solution YA of the compound is given in step S22. Then, the head 24 is actuated by this signal, and the solution YA of compound 1 stored in the compound solution plate 17 is supplied to the liquid flow path 45 by the pipette tip 50. Subsequently, the assay data G1 and reference data G2 are acquired in step S23, and the data G3 of the amount of binding is determined by correcting the assay data G1 with the reference data in step S24. The data G3 of the amount of binding is stored in the memory 60D in step S25 in correspondence with the acquired time.

The data G3 of the amount of binding as well as the assay time of a given compound are stored by the above-mentioned bonding amount assay process.

The washing process is implemented in step S30 in Fig. 9. An instruction signal for flow-in of the buffer solution is given in step S32. The head 24 is actuated by this signal, and the buffer solution stored in the buffer liquid plate 18 is supplied to the liquid flow path 45 by one of the pipette tips 50 while the buffer solution is drawn by the other pipette tip 50 to permit the buffer liquid to flow in the liquid flow path 45. Subsequently, whether a predetermined amount of the buffer liquid has been supplied or not is judged in step S33. The predetermined amount as used herein means an amount sufficient for dissociating the biologically active substance D from compound A, and the amount may be prescribed by the flow-in time of the buffer liquid. A signal for stopping the supply of the buffer liquid is given in step S34 when the predetermined amount of the buffer liquid is judged to have been supplied. Supply of the buffer liquid is stopped by this signal, and washing of the liquid flow path 45 is completed.

Compound A is removed from the biologically active substance D by the above-mentioned washing process, and a state where assay of the succeeding compound solution YA is possible is attained.

Whether or not the N-th bounding amount assay process has been completed, that is whether or not all the assay/washing steps repeatedly applied to one biologically active substance D have been completed, is judged in step S40 shown in Fig. 9. When all the assay/washing steps have not been completed, whether or not a predetermined time from the preceding activity assay process has been passed is judged. The predetermined time as used herein refers to

a time interval determined in advance for implementing the activity assay process. If the predetermined time is judged to have been passed, the process returns to step S10 and the activity assay process is implemented again. On the other hand, if the predetermined time is judged not to have passed, the amount of binding of the succeeding compound A is measured in step S20.

When all the assay/washing steps are judged to have been completed in step S40, the activity assay process is repeated in step S44. The activity assay process in this step is the same as the activity assay process shown in Fig. 10 and implemented in step S10.

The correction process is applied in step S50. The data G3 of the amount of binding between all the compounds A and the biologically active substance D are corrected with reference to the relationships to the activity K of the biologically active substance D in the correction process.

The activity K of the biologically active substance D stored in the memory 60D is read in step S52 in the correction process as shown in Fig. 13, and an approximated linear line showing the relationship between the assay time and the biologically active substance D is calculated in step S54. For example, when activities K1 to KN are obtained in the first to N-th assays of the activity as shown in Fig. 14, the approximated linear line may be determined by a least-square method. The approximated linear line M corresponds to the relationship of activity change indicating the time-dependent change of the activity K.

The data G3 of the amount of binding with respect to each compound A is corrected in step S56 based on the approximated linear line M to calculate the correcting bonding amount data HG3. The correcting bonding amount data HG3 may be determined by equation 1 by assuming that the activity at an assay time TO of the first activity assay is represented by KO, and the activity at the time when the data G3 of the amount of binding is represented by KT;

$$HG3 = G3/(KT/KO) \qquad (1)$$

This process is completed in step S58 by storing the data HG3 of the amount of binding after the correction with respect to each compound A. Correction of the data G3 of the amount of binding permits a more precise amount of binding to be relatively determined in relation to other compounds A.

Subsequently, a sorting process is implemented in step S60 shown in Fig. 9. In the sorting process, the correcting bonding amount data HG3 is read from the memory 60D in step S62 as shown in Fig. 15, and whether or not the read correcting bonding amount data HG3 is larger than a predetermined threshold level is judged in step S63. When the correcting bonding amount data HG3 is larger than the threshold level, the compound A is extracted as a candidate compound in step S64. Whether or not a succeeding compound A exists is judged in step S65, and the above-mentioned process is repeated by returning to step S62 when a succeeding compound is judged to exist. This process is completed when no succeeding compound A is found since sorting of all the compounds A has been completed.

More precise sorting of the compound is possible according to this exemplary embodiment since the compound A specifically bonded to the biologically active substance D is sorted based on the correcting bonding amount data HG3.

In this exemplary embodiment, the activity assay process is carried out for one biologically active substance D at first before the first assay of the amount of binding, each time thereafter that the predetermined amount of time elapses, and after the last assay of the amount of binding. However, the activity may be assayed at least twice for one biologically active substance D in order to determine the relationship of activity change based on the activities K obtained by two times that the activity assay processing is carried out. However, it is preferable that the activity is assayed at least before the first assay of the amount of binding and after the last assay of the amount of binding in order to obtain a more precise relationship of activity change.

Further, the relationship of activity change is not always required to be an approximated liner line, and may be a curve based on the activities K obtained from a plurality of activity assays.

While the data G3 of the amount of binding are corrected based on the time-dependent change of the activity in this exemplary embodiment, a relationship of activity change based on the number of times that the assay/washing process is carried out may be determined, and the data G3 of the amount of binding may be corrected based on this relationship of activity change.

While the biosensor was described using the surface plasmon sensor as an example, the biosensor is not restricted to the surface plasmon sensor. Other examples available in the invention include technologies using various biosensors such as a quartz oscillator micro-balance (QCM) method and an optical assay method using functionalized surfaces of particles from colloid particles to ultra-fine particles.

An example of other biosensors taking advantage of decay of the total reflection is a leaky mode detector. The leaky mode sensor comprises a dielectric and a thin film composed of a clad layer and an optical waveguide layer sequentially laminated on the dielectric, wherein one surface of this thin film serves as a sensor surface and the other surface serves as a light incidence surface. When a light beam impinges on the light incidence surface so as to satisfy a total reflection condition, a part of the light enters into the optical waveguide layer by permeating through the clad

layer. When a waveguide mode is excited in the optical waveguide layer, the reflection light on the light incidence surface is largely decayed. The incident angle by which the waveguide mode is excited changes depending on the refraction index of the medium on the sensor, as in the surface plasmon resonance angle does. The reaction on the sensor surface is measured by detecting the decay of the reflection light.

EXAMPLES

Examples of practical screening using the biosensor 10 described in the exemplary embodiments will be described below.

Example 1

(1) Preparation of the assay chip (sensor stick)

A dielectric block 42 on which gold with a thickness of 50 nm is deposited by vapor deposition as a metal film 57 was treated with an ozone cleaning system (trade name; Model-208 UV, manufactured by TECHNOVISION INC.), and a 1.0 mM solution of 16-hydroxy-1-hexadecane thiol dissolved in ethanol/water (80:20 in volume ratio) was added so that the solution is in contact with the metal film. The metal film was subjected to surface treatment at 25°C for 1 hour. The metal film was washed with ethanol five times, with a mixed solvent of ethanol/water once and with water 5 times.

Then, an epichlorohydrin solution (10% by mass in a 1:1 mixed solvent of 0.4 M aqueous sodium hydroxide and diethyleneglycol dimethylether) was made to contact the surface coated with 16-hydroxy-1-hexadecane thiol, and the reaction was allowed to proceed for 4 hours in a shaking incubator at 25°C. The surface was washed with ethanol twice, with water 5 times. Subsequently, 4.5 ml of 1 M aqueous sodium hydroxide was added to 40.5 ml of 25% aqueous solution of dextran (T500, manufactured by Pharmacia), and the solution was made to contact the epichlorohydrin-treated surface. The dielectric block was then incubated in a shaking incubator for 20 hours at 25°C, and the surface of the block was washed with water warmed at 50°C 10 times. Subsequently, a mixture prepared by dissolving 3.5 g of bromoacetic acid in 27 g of 2 M aqueous sodium hydroxide was made to contact the dextran-treated surface, and the block was incubated in a shaking incubator for 16 hours at 25°C. The surface was washed with water, and the same bromoacetic acid treatment was repeated once.

(2) Fixing of protein

p38 MAP kinase (#559324, manufactured by CALBIOCHEM) was attached on the hydrogel-coated assay chip prepared in (1) by the following procedure.

A protein solution containing 100 μg/ml of p3 8 MAP kinase and 10 μM of compound 1 was prepared using an acetate buffer (pH 5.5). The composition of a running buffer was as follows: 50 mM phosphate buffer (pH 7.2)/150 mM NaCl/3.4 mM EDTA.

The running buffer was added to the hydrogel-coated chip for measuring a baseline signal. Then, a mixed solution of 1-ethyl-2,3-dimethylaminopropyl carbodiimide (400 mM) and N-hydroxy succinimide (100 mM) was added to the chip, which was washed with the running buffer alter allowing it to stand for 15 minutes. The prepared protein solution was added to the chip, which was washed with a PBS buffer (pH 7.4) after allowing it to stand for 20 minutes. An ethanolamine HCl solution (1 M, pH 8.5) was further added to the chip, which was washed with the running buffer three times after allowing it to stand for 15 minutes. The change of the signal from the first baseline signal was defined to be the amount of fixing (RU) of p38 MAP kinase. The amount of fixing of p38 MAP kinase was 3400 RU.

(3) Assay of bonding of the compound

The assay chip on which the protein was attached was attached to a surface plasmon resonance measuring apparatus, and the amount of binding of compound 1 was assayed as follows. Compound 1 used herein is a compound that has been known to inhibit p38 MAP kinase activity.

A running buffer with the following composition was prepared: 50 mM phosphate buffer (pH 7.2)/150 mM NaCl/3.4 mM EDTA/DMSO 5% by volume.

Then, compound 1 is dissolved in the running buffer at a concentration of 10 μM. The running buffer was added to the hydrogen-coated chip for measuring a baseline signal. Subsequently, the solution of the compound prepared was added to the chip, and the change of the signal after allowing the chip to stand for 2 minutes was defined to be the amount of binding (RU) of each compound. The running buffer was finally added to the chip, which was washed three times for 30 seconds.

This operation was repeated 44 times on the surface on which the same protein was attached. The results of

measurement are shown in Fig. 17. The amount of first bonding was 14.1 RU while the amount of 44-th bonding was 11.5 RU, and the amount of binding was decreased about 18% during this procedure.

[compound 1]

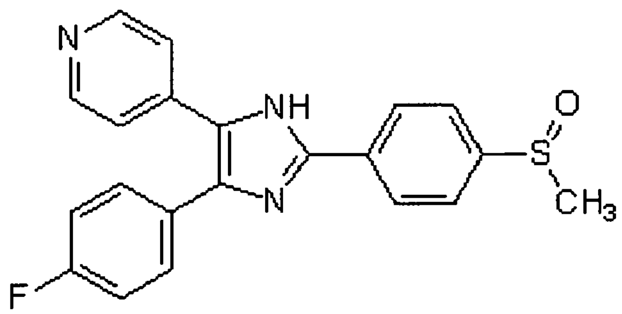

Example 2

(1) Activity assay of protein

The activity of p38 MAP kinase attached on the surface in Example 1 was assayed by the following method before the assay of the amount of binding of compound 1 and after 44 times of the assay of the amount of binding as described in Example 1.

Surfaces on which p38 MAP kinase was attached and not attached, respectively, were prepared by the method in Example 1. A kinase assay buffer (50 μl; 50 mM Tris HCl buffer pH7.4, 20 μM myelin basic protein, 10 μM ATP, 20 mM MgCl2) was added to each surface. p38 MAP kinase (25 ng, 50 ng, 100 ng and 200 ng) was prepared at the same time, and 50 μl of the kinase assay buffer was added to the protein used for correction by the following procedure. After adding the kinase assay buffer to the protein, the solution was incubated at room temperature for 1 hour, and the reaction solution was transferred to a 96 well plate. KinaseGlo reagent (trade name; manufactured by Promega, 50 μl) was added to each well, and the solution was incubated at room temperature for 10 minutes. Residual ATP-dependent light emission was measured with LAS 1000 (trade name; manufactured by FUJIFILM). The emission from the surface on which no protein is attached is used as a background signal, and the intensity of light emission from attached p38 MAP kinase was calculated.

The activity of attached p38 MAP kinase was calculated relative to 100% of the activity of p38 MAP kinase for correction using the amount of fixing of p38 MAP kinase calculated by the SPR assay by the method in example 1 and the intensity of light emission of the attached p38 MAP kinase.

(Calculation equation)

Activity (%) of attached p38 MAP kinase = [(intensity of light emission of attached p38 MAP kinase)/(intensity of light emission of p38 MAP kinase for correction corresponding to the same amount of attached p38 MAP kinase) x 100

A linear line for correcting the amount of binding at each assay was determined based on the p38 MAP kinase activities before the assay of bonding and after 44-th assay of boding, and the amount of binding could be corrected based on the linear line for correction.

The relative activity of p38 MAP kinase before the assay of the amount of binding of compound 1 was 45%, while the relative activity of p38 MAP kinase after 44-th assay of the amount of binding of compound 1 was 35%. The activity was decreased about 22% during the interval of two assays, and it was shown that this decrease approximately matches the extent of decrease of the amount of binding of compound 1 in Example 1.

The result indicates that it is quite effective to assay the activity of the protein before and after bonding the compound, and to correct the amount of binding of the compound using the extent of decrease when the amount of binding of the compound is repeatedly assayed on the surface on which the same protein is attached.

Since the activity of the biologically active substance is considered to be changing, not constant, during the

interval when the assay/washing process is repeated, the biologically active substance should be sorted by taking this activity change into consideration.

The object of the invention performed by taking the above-mentioned facts into consideration is to provide a screening method capable of more precisely sorting the compound specifically bonded to the biologically active substance and a screening apparatus.

The compound specifically bonded to the biologically active substance may be more precisely sorted by the invention configured as described above.

All publications, and technical standards mentioned in this specification are herein incorporated by reference to the same extent as if each individual publication, patent application, or technical standard was specifically and individually indicated to be incorporated by reference.

## Claims

**1.** A method for screening a specified compound, the method comprising:

repeatedly applying an assay/washing process to the same biologically active substance for each of different plural compounds, the assay/washing process comprising supplying a compound to an attached biologically active substance, assaying the amount of binding between the biologically active substance and the compound, and washing the compound supplied to the biologically active substance after the assay; and
repeating an activity assay for detecting the activity of the biologically active substance at least twice during the interval from before starting of the first assay/washing process to after completion of the last assay/washing process,
wherein a relationship of activity change indicating the activity change of the biologically active substance is determined based on the activity of the biologically active substance obtained by the activity assay, a corrected amount of binding is determined by correcting the amount of binding between the biologically active substance and the compound based on the relationship of activity change, and a compound specific to the biologically active substance is sorted based on the corrected amount of binding.

**2.** The screening method of Claim 1, wherein the activity is assayed at least before starting of the first assay/washing process and after completion of the last assay/washing process.

**3.** The screening method of Claim 1, wherein the relationship of activity change indicates time-dependent change of the activity, and the corrected amount of binding H is determined by an equation of $H = S/(KT/KN)$, where the activity before starting the assay is represented by a fresh activity KN, the activity at the time of assay is represented by an activity KT, and the amount of binding obtained by the assay is represented by an assayed amount of binding S.

**4.** The screening method of Claim 2, wherein the relationship of activity change indicates time-dependent change of the activity, and the corrected amount of binding H is determined by an equation of $H = S/(KT/KN)$, where the activity before starting the assay is represented by a fresh activity KN, the activity at the time of assay is represented by an activity KT, and the amount of binding obtained by the assay is represented by an assayed amount of binding S.

**5.** A screening apparatus comprising: an assay chip on which a biologically active substance is attached and having formed thereon a liquid pool capable of pooling a liquid on the biologically active substance; an assay/washing unit for repeating an assay/washing process for supplying a compound to the liquid pool and washing the compound supplied to the biologically active substance after assaying the amount of binding between the biologically active substance and the compound for each of different plural compounds with respect to the same biologically active substance; an activity assay unit for detecting the activity of the biologically active substance at least twice during the interval from before starting of the first assay/washing process to after completion of the last assay/washing process; an activity change relationship calculating unit for determining a relationship of activity change that indicates the activity change of the biologically active substance based on the activity of the biologically active substance detected by the activity assay unit; a bonding amount correction unit for determining a corrected amount of binding by correcting the amount of binding between the biologically active substance and the compound based on the relationship of activity change; and a sorting unit for sorting a compound specific to the biologically active substance based on the corrected amount of binding.

**6.** The screening apparatus of Claim 5, further comprising a retrieval unit for retrieving the liquid supplied to the liquid pool.

F I G. 1

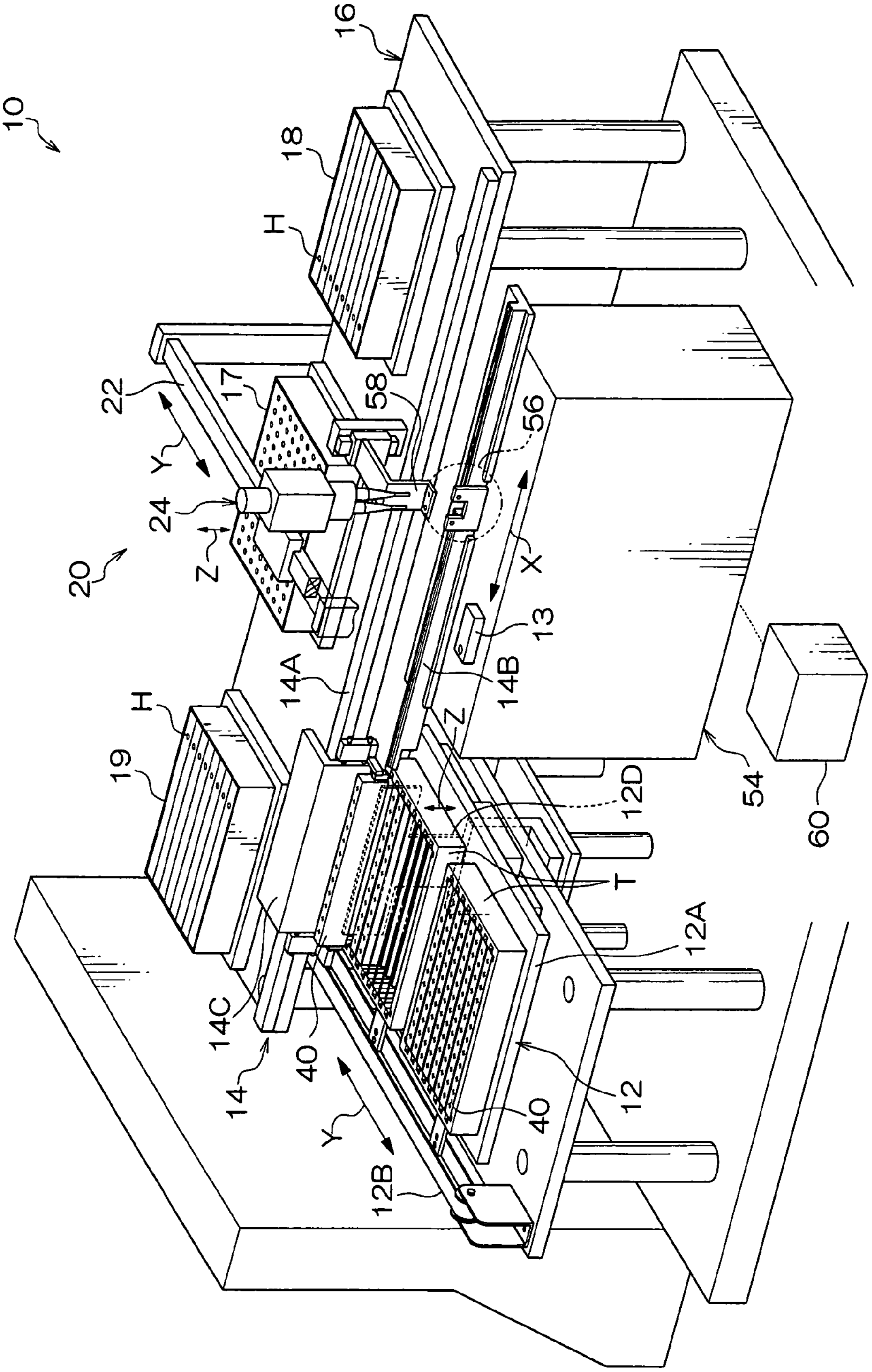
10
16
18
H
22
17
58
56
Y
24
Z
20
X
13
14A
14B
H
19
12D
54
60
Z
T
12A
14C
14
40
40
12
Y
12B

FIG. 2

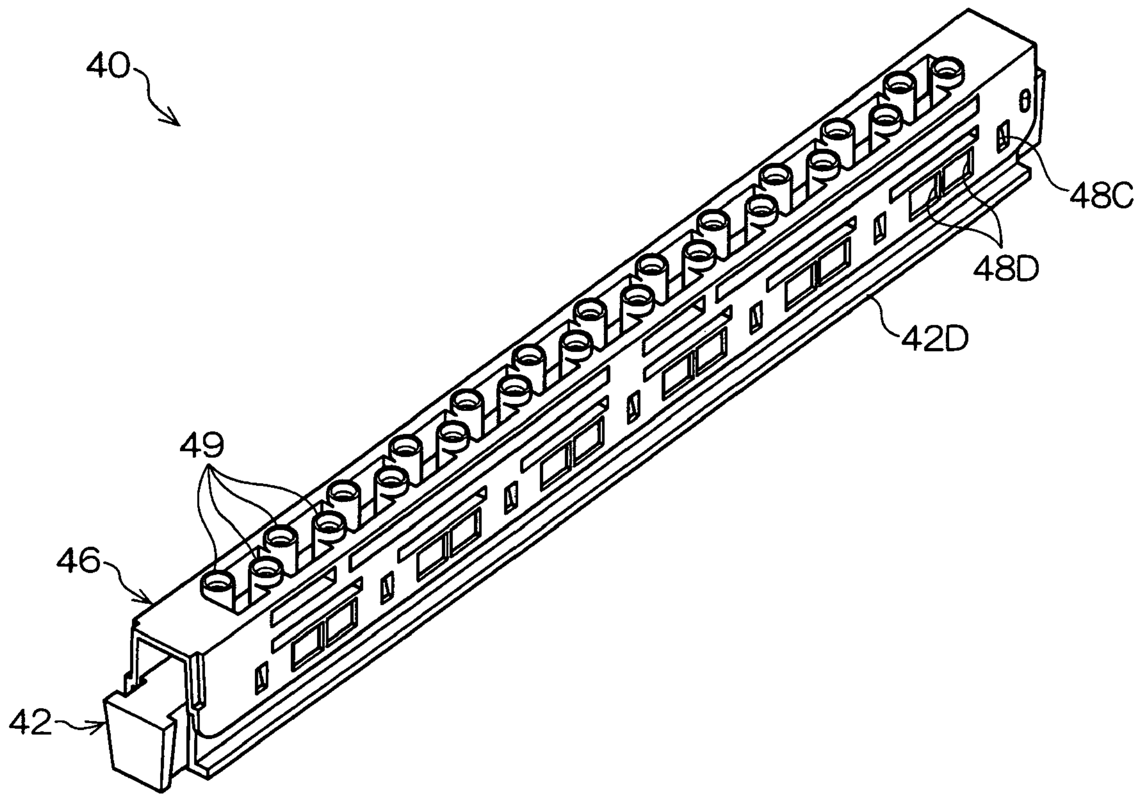

F I G. 3

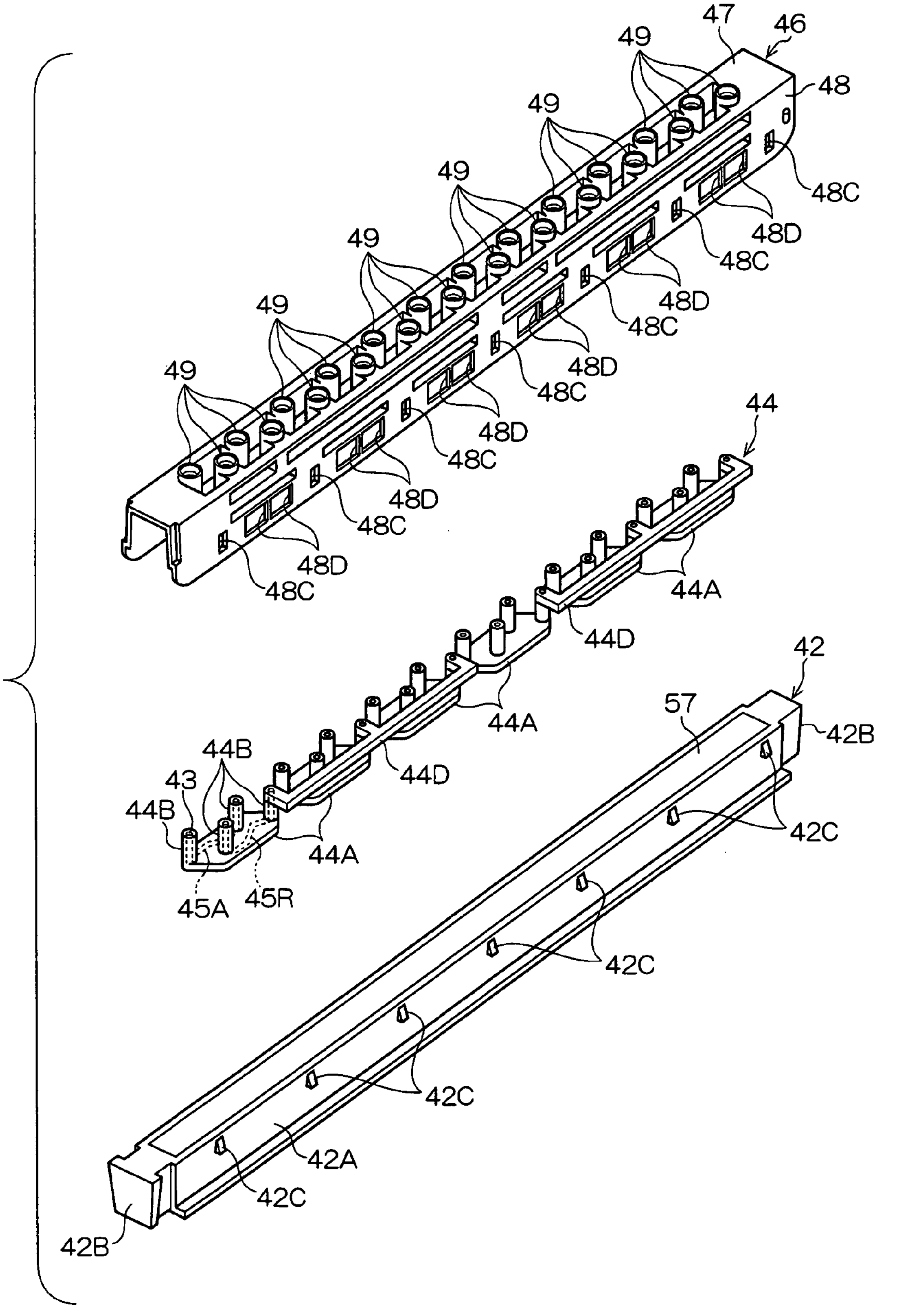

FIG. 4

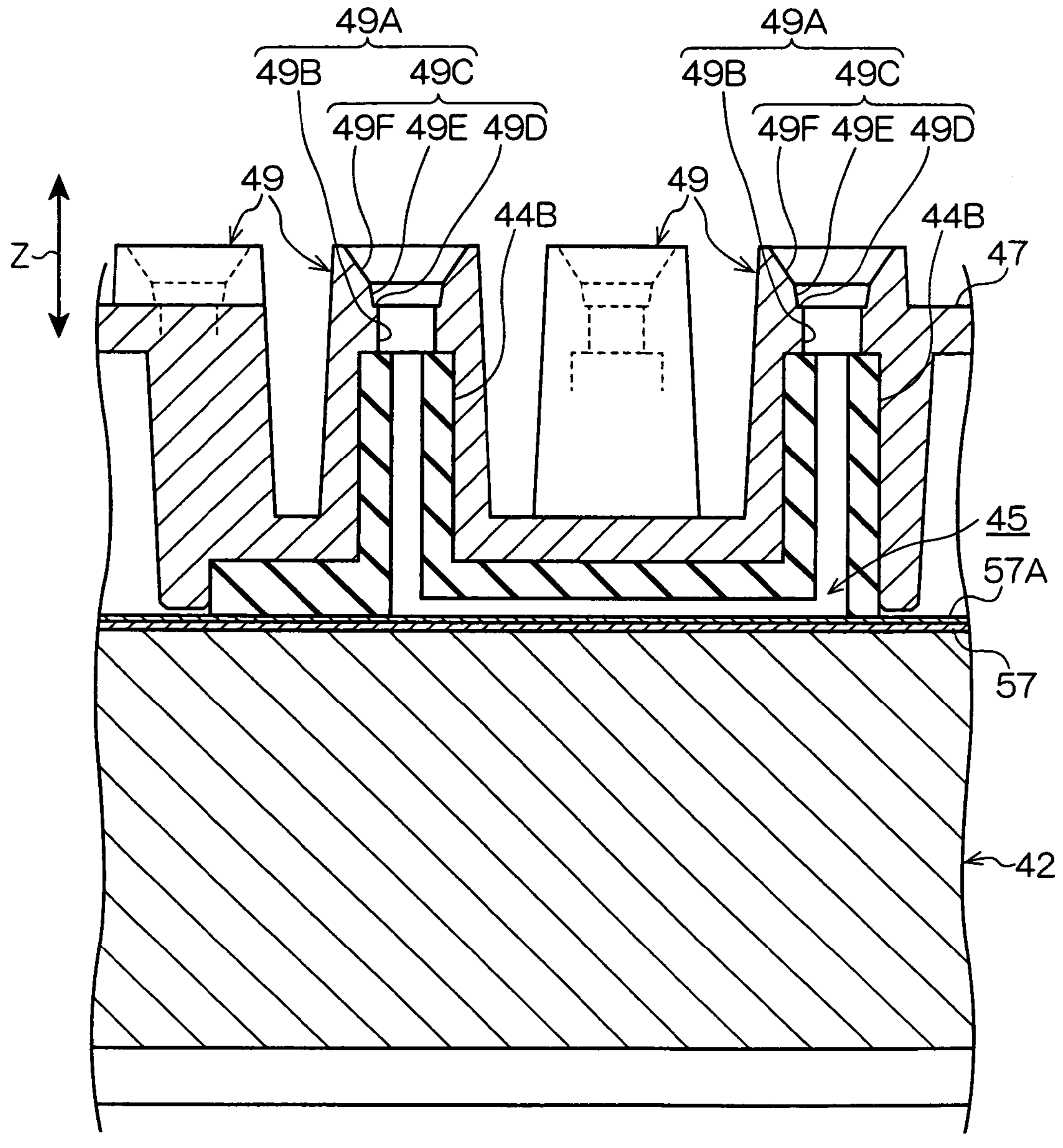
49A
49B
49C
49F 49E 49D
49
44B
Z
49A
49B
49C
49F 49E 49D
49
44B
47
45
57A
57
42

F I G. 5

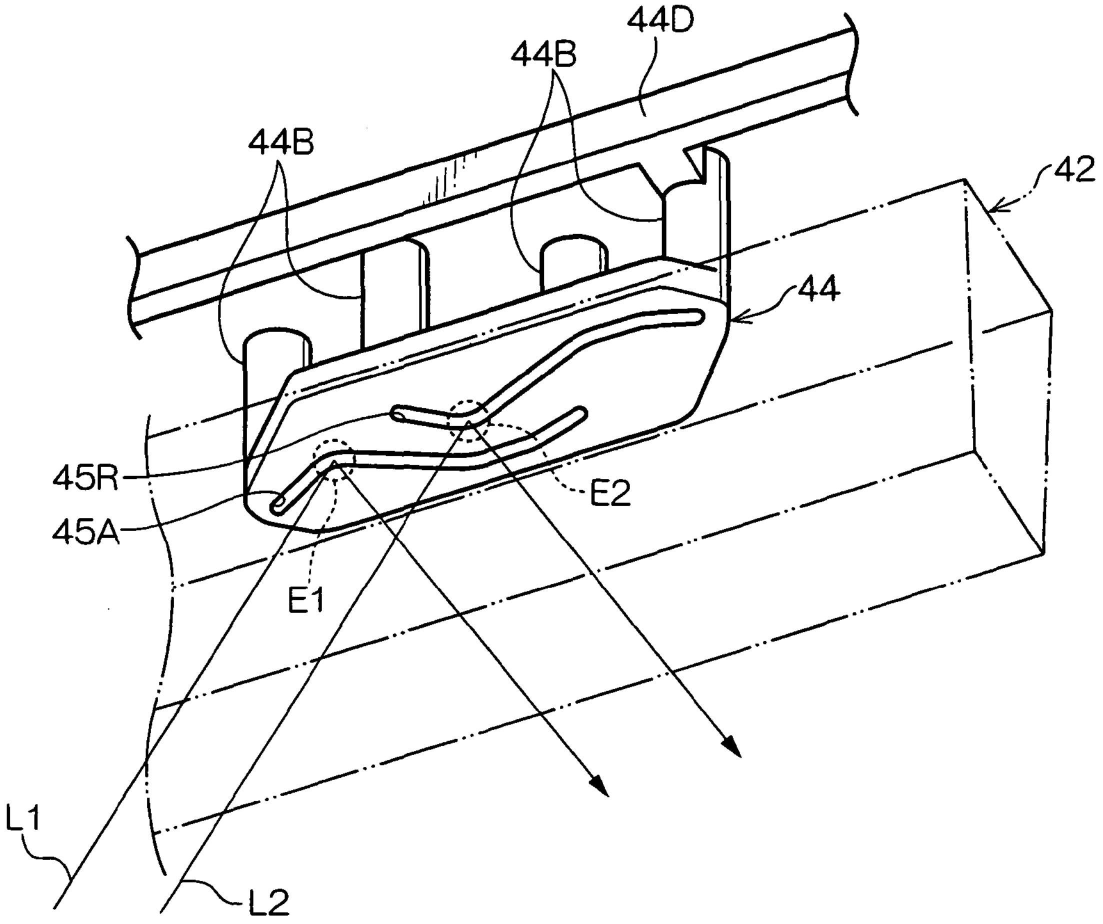

F I G. 6

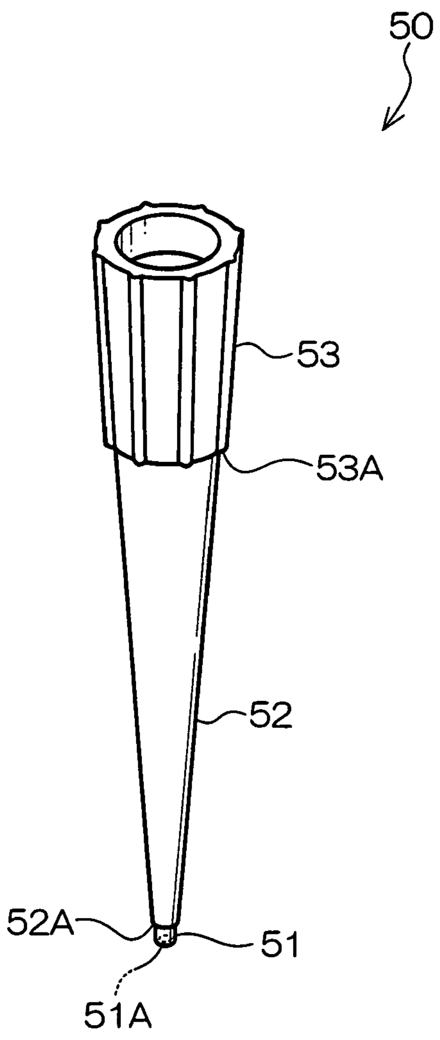

FIG. 7

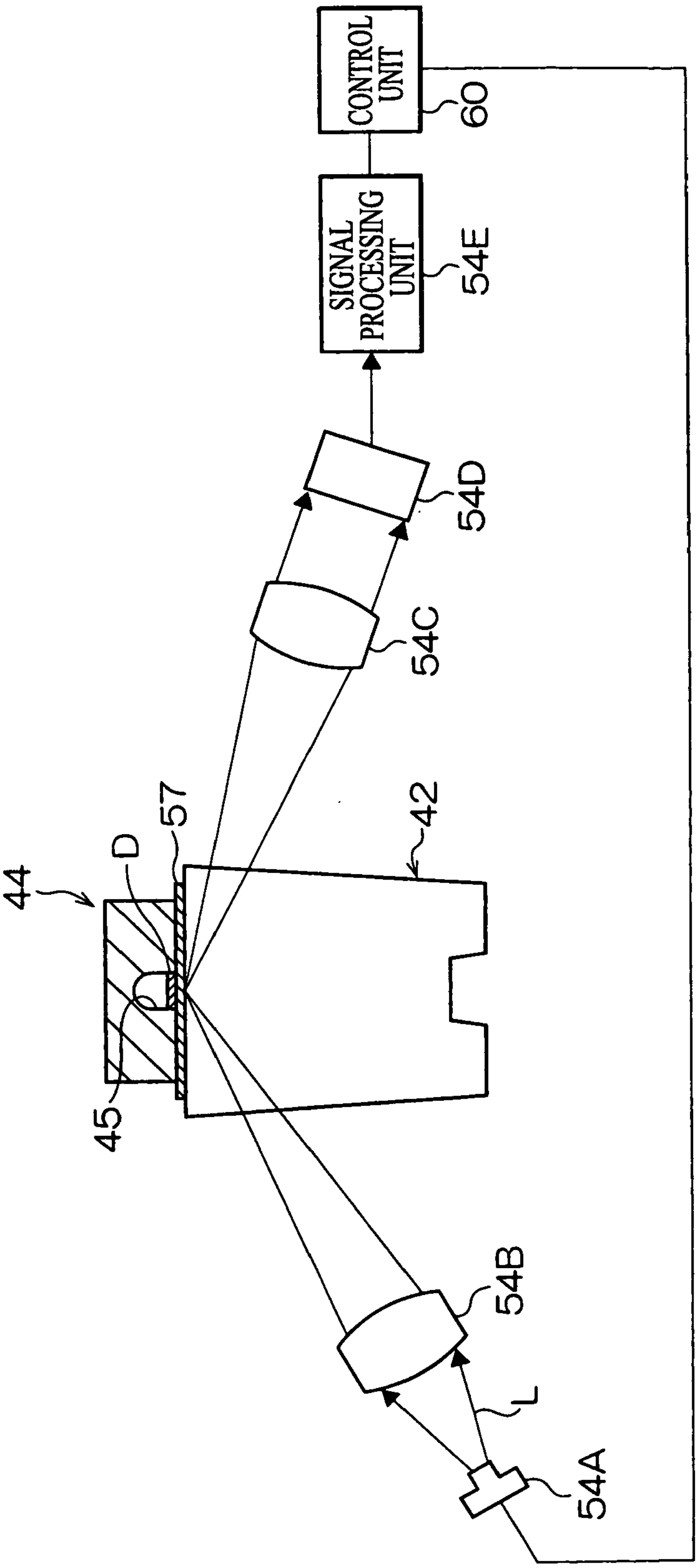
CONTROL UNIT
60
SIGNAL PROCESSING UNIT
54E
54D
54C
42
57
D
44
45
54B
L
54A

F I G. 8

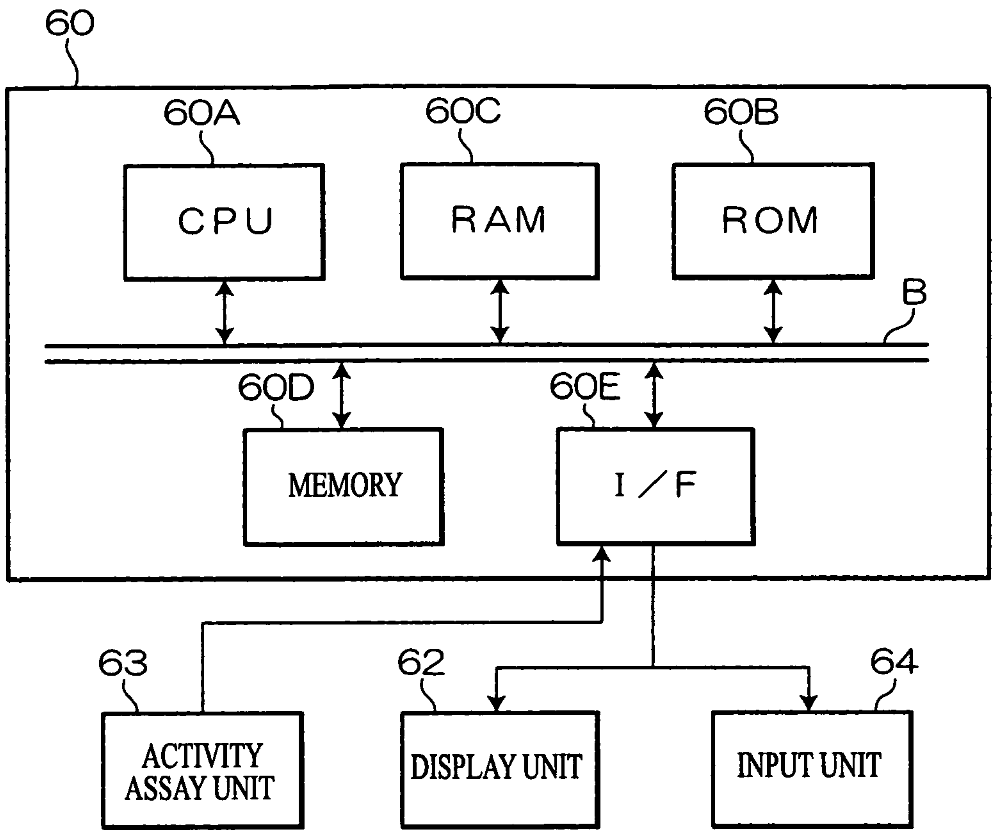
60
60A
60C
60B
CPU
RAM
ROM
B
60D
60E
MEMORY
I／F
63
62
64
ACTIVITY ASSAY UNIT
DISPLAY UNIT
INPUT UNIT

FIG. 9

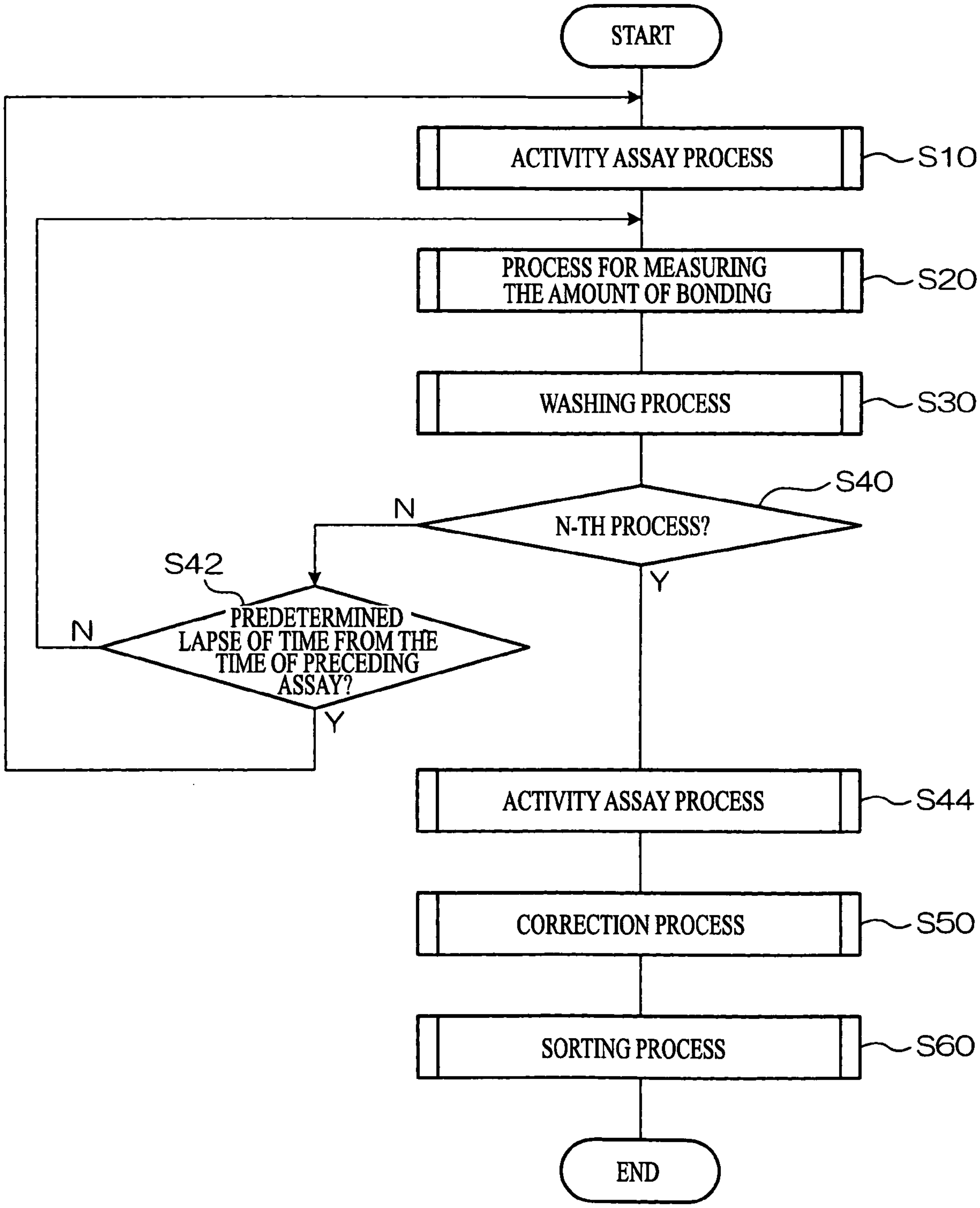
START
ACTIVITY ASSAY PROCESS
S10
PROCESS FOR MEASURING THE AMOUNT OF BONDING
S20
WASHING PROCESS
S30
N-TH PROCESS?
S40
N
Y
PREDETERMINED LAPSE OF TIME FROM THE TIME OF PRECEDING ASSAY?
S42
N
Y
ACTIVITY ASSAY PROCESS
S44
CORRECTION PROCESS
S50
SORTING PROCESS
S60
END

F I G. 1 0

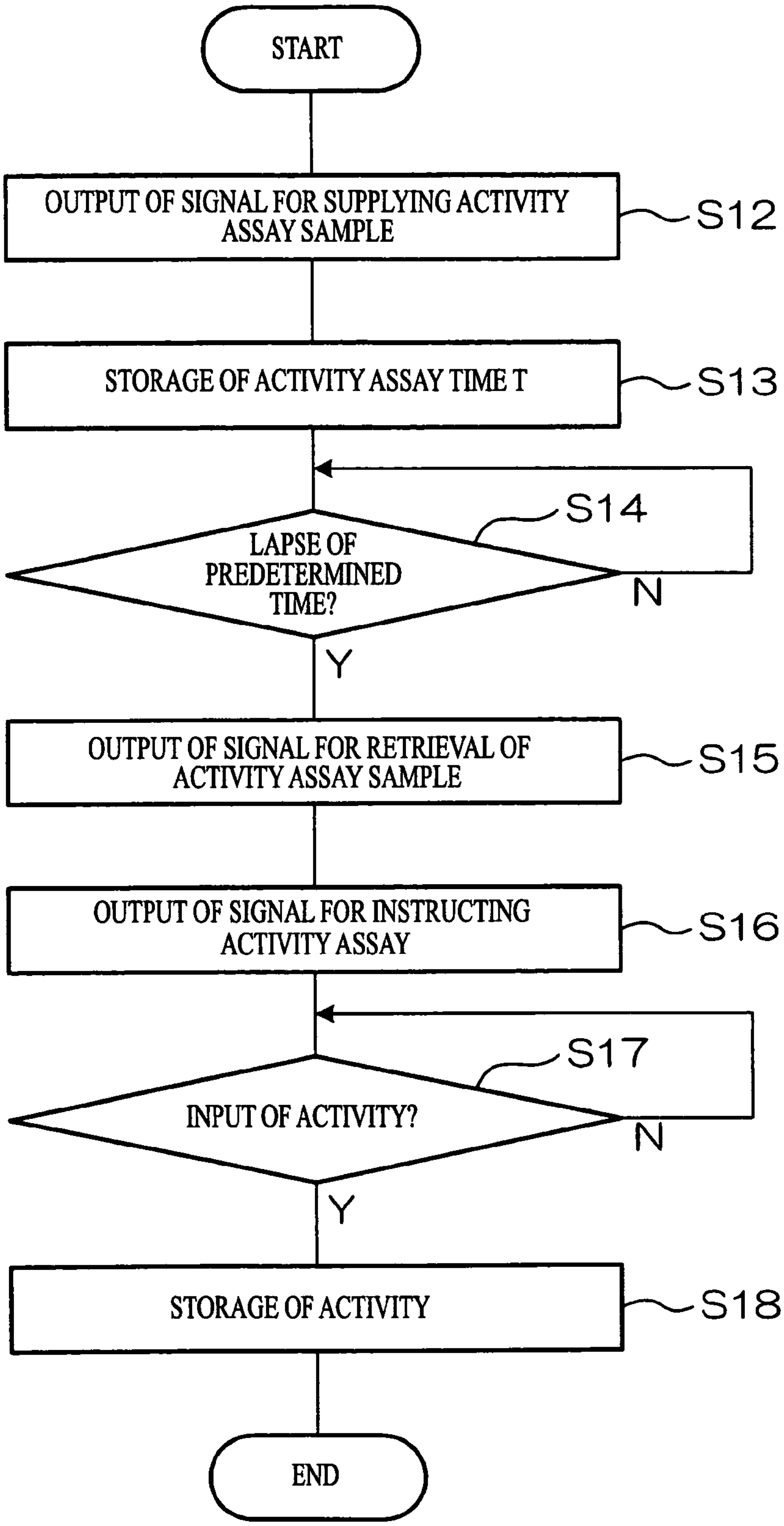
START
OUTPUT OF SIGNAL FOR SUPPLYING ACTIVITY ASSAY SAMPLE
S12
STORAGE OF ACTIVITY ASSAY TIME T
S13
LAPSE OF PREDETERMINED TIME?
S14
N
Y
OUTPUT OF SIGNAL FOR RETRIEVAL OF ACTIVITY ASSAY SAMPLE
S15
OUTPUT OF SIGNAL FOR INSTRUCTING ACTIVITY ASSAY
S16
INPUT OF ACTIVITY?
S17
N
Y
STORAGE OF ACTIVITY
S18
END

FIG. 11

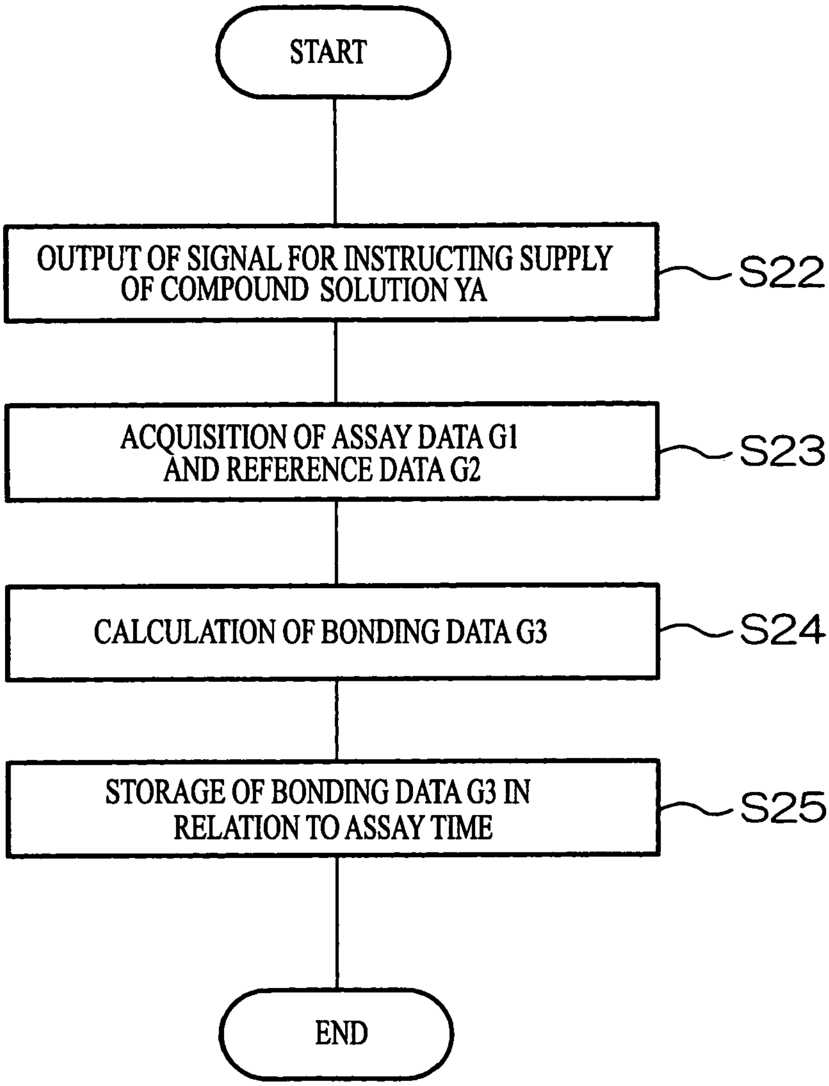
START
OUTPUT OF SIGNAL FOR INSTRUCTING SUPPLY OF COMPOUND SOLUTION YA
S22
ACQUISITION OF ASSAY DATA G1 AND REFERENCE DATA G2
S23
CALCULATION OF BONDING DATA G3
S24
STORAGE OF BONDING DATA G3 IN RELATION TO ASSAY TIME
S25
END

FIG. 12

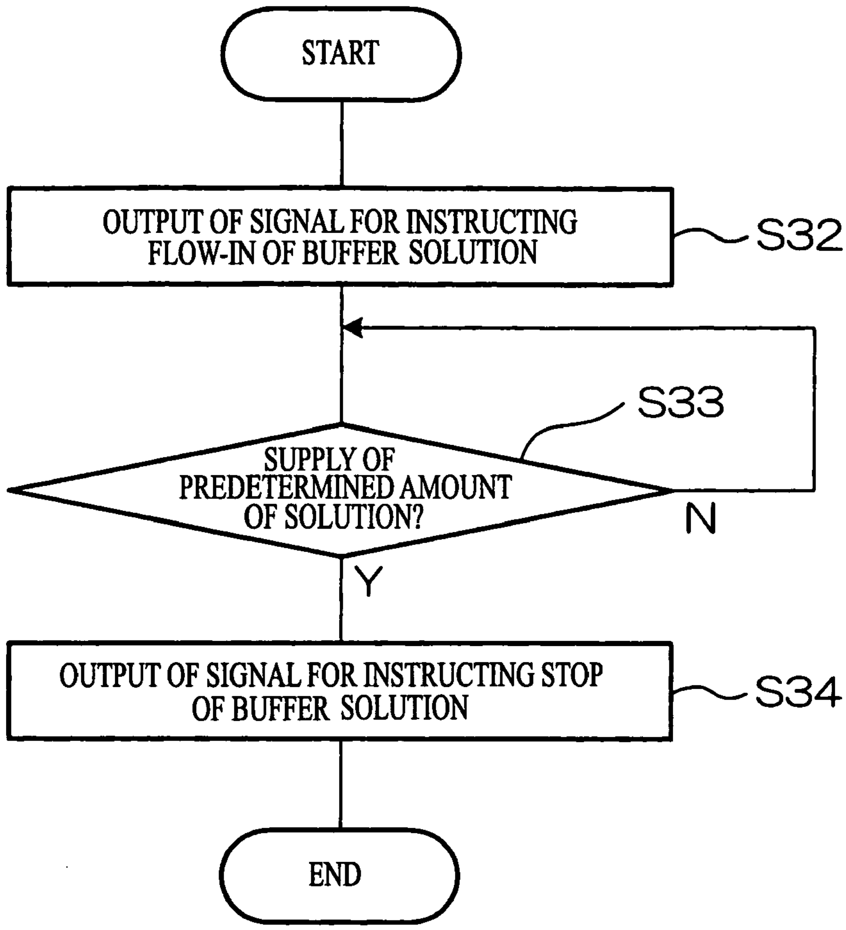
START
OUTPUT OF SIGNAL FOR INSTRUCTING FLOW-IN OF BUFFER SOLUTION
S32
S33
SUPPLY OF PREDETERMINED AMOUNT OF SOLUTION?
N
Y
OUTPUT OF SIGNAL FOR INSTRUCTING STOP OF BUFFER SOLUTION
S34
END

FIG. 13

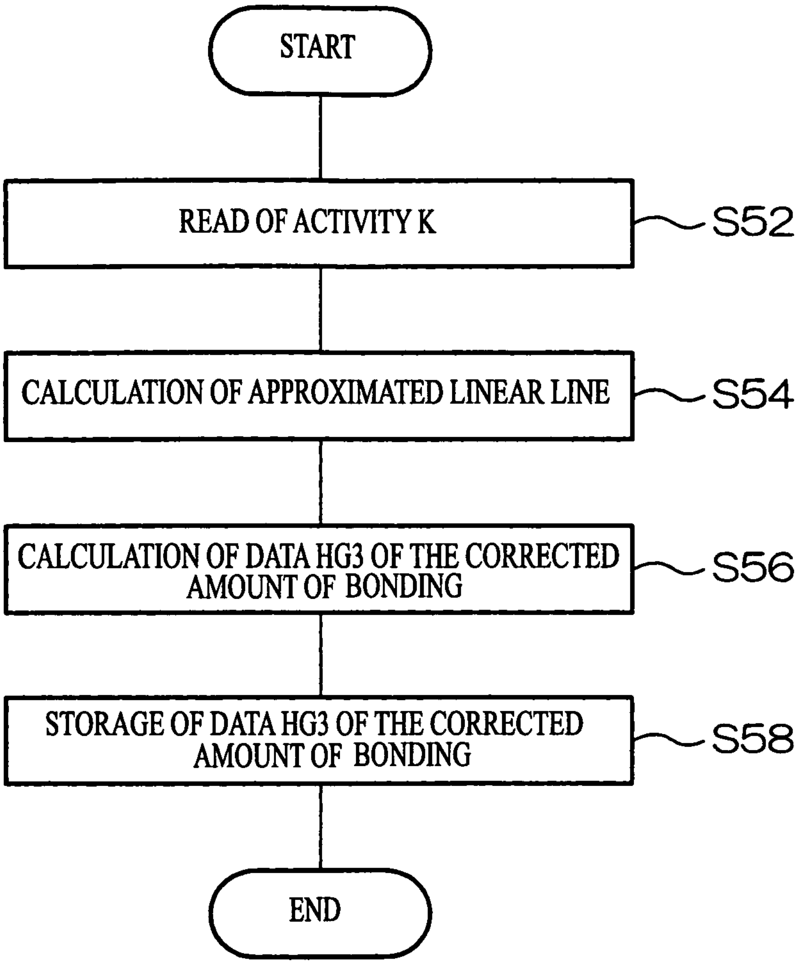
START
READ OF ACTIVITY K
S52
CALCULATION OF APPROXIMATED LINEAR LINE
S54
CALCULATION OF DATA HG3 OF THE CORRECTED AMOUNT OF BONDING
S56
STORAGE OF DATA HG3 OF THE CORRECTED AMOUNT OF BONDING
S58
END

FIG. 14

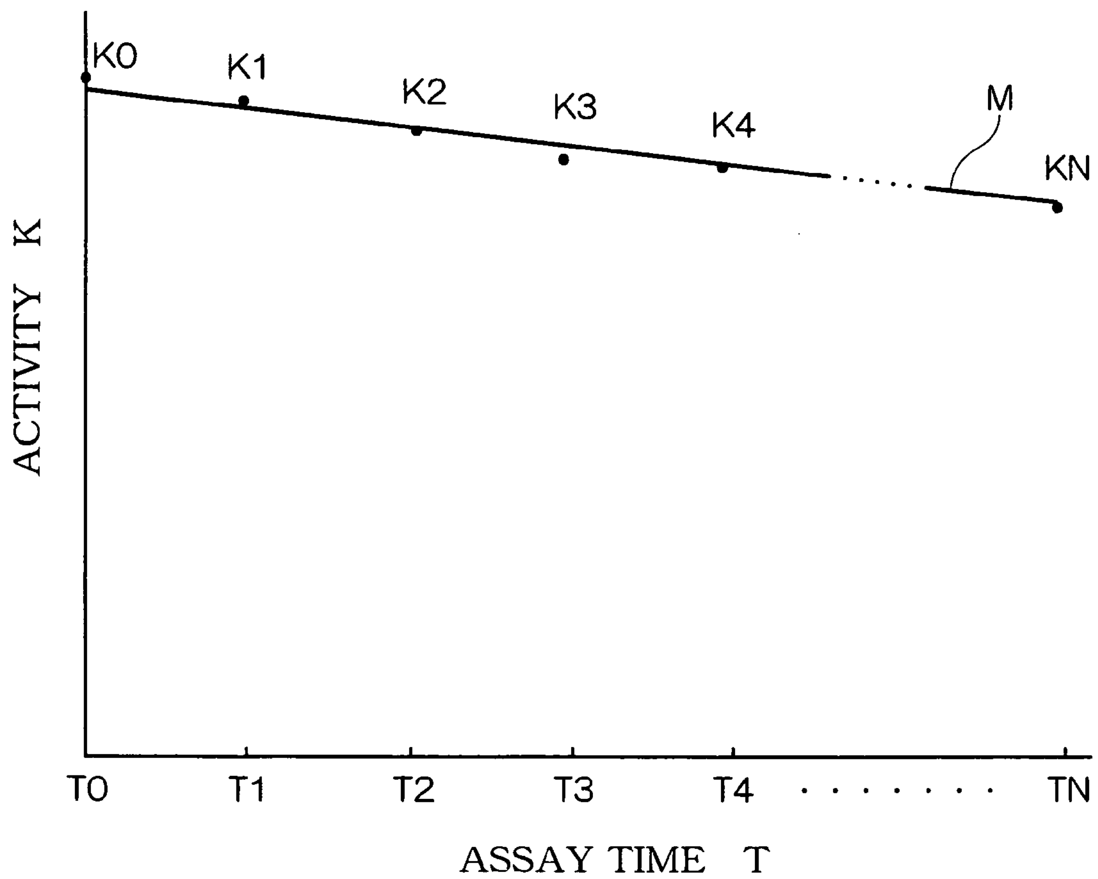

FIG. 15

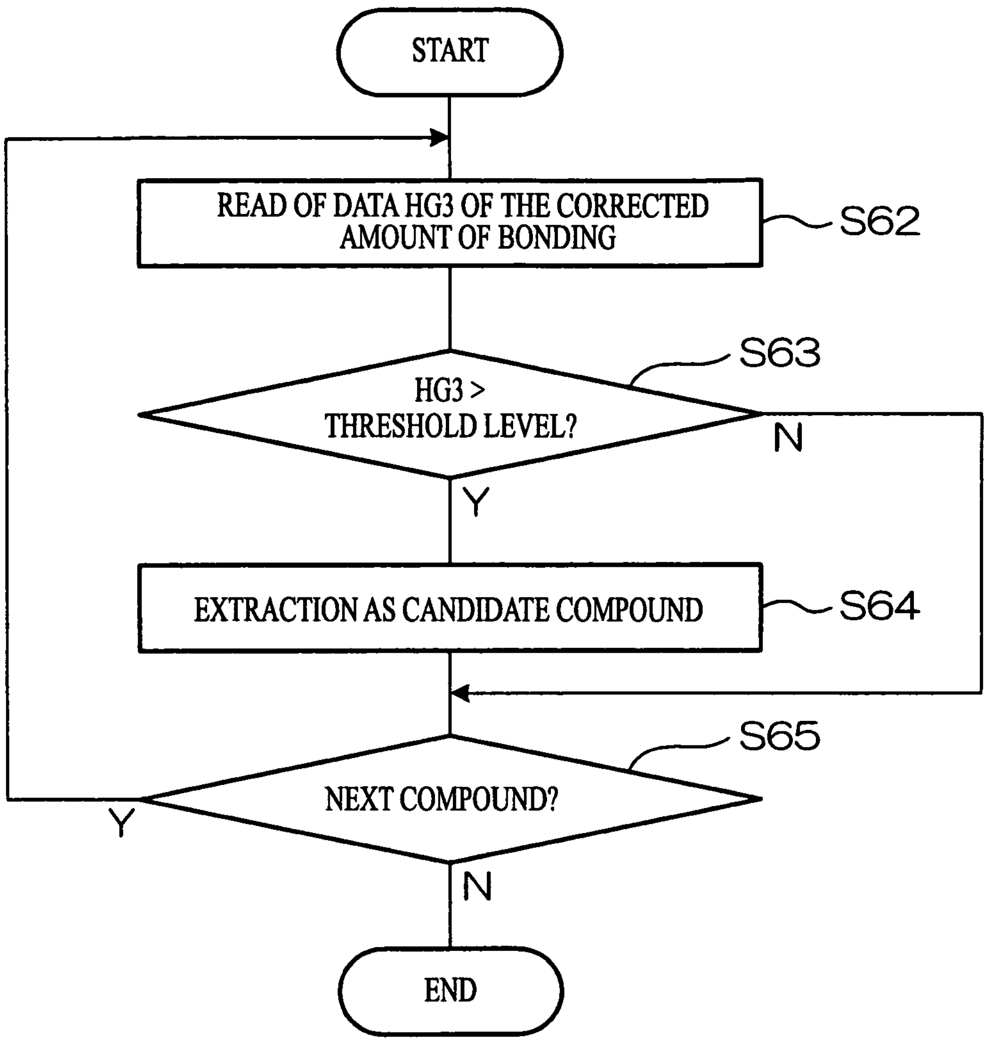
START
READ OF DATA HG3 OF THE CORRECTED AMOUNT OF BONDING
S62
S63
HG3 > THRESHOLD LEVEL?
N
Y
EXTRACTION AS CANDIDATE COMPOUND
S64
S65
NEXT COMPOUND?
Y
N
END

FIG. 16

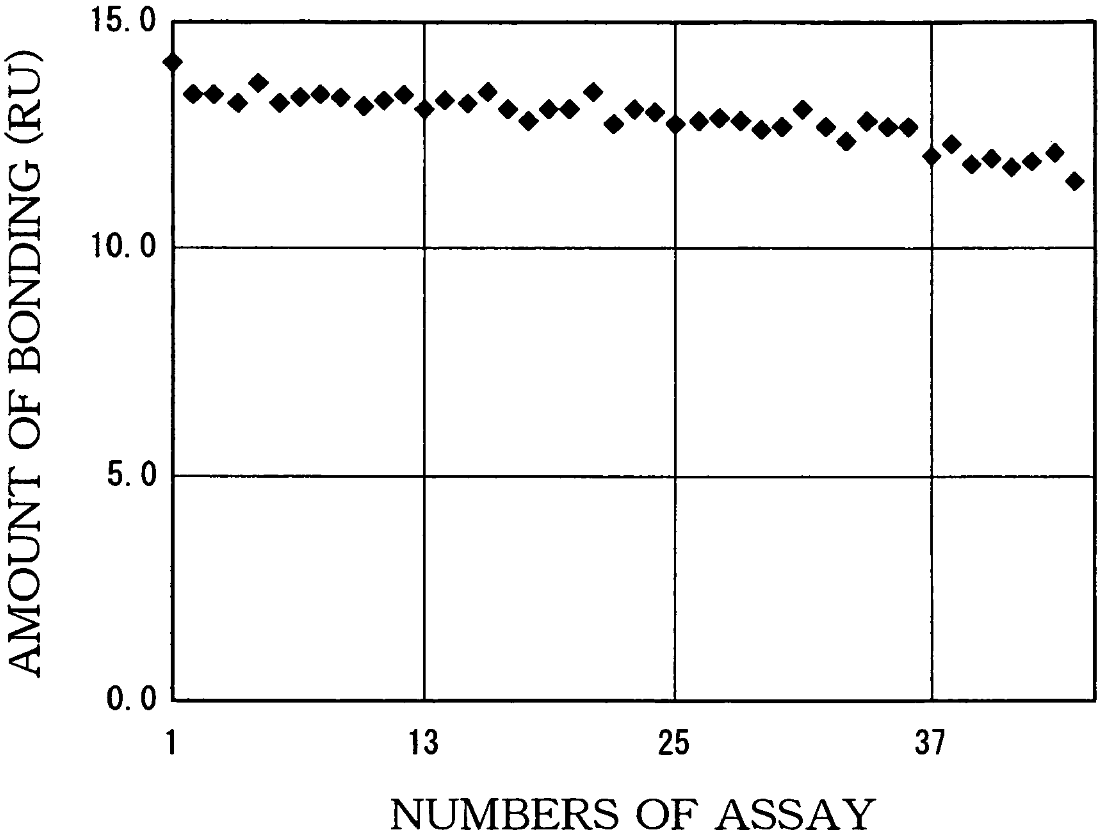

F I G. 1 7

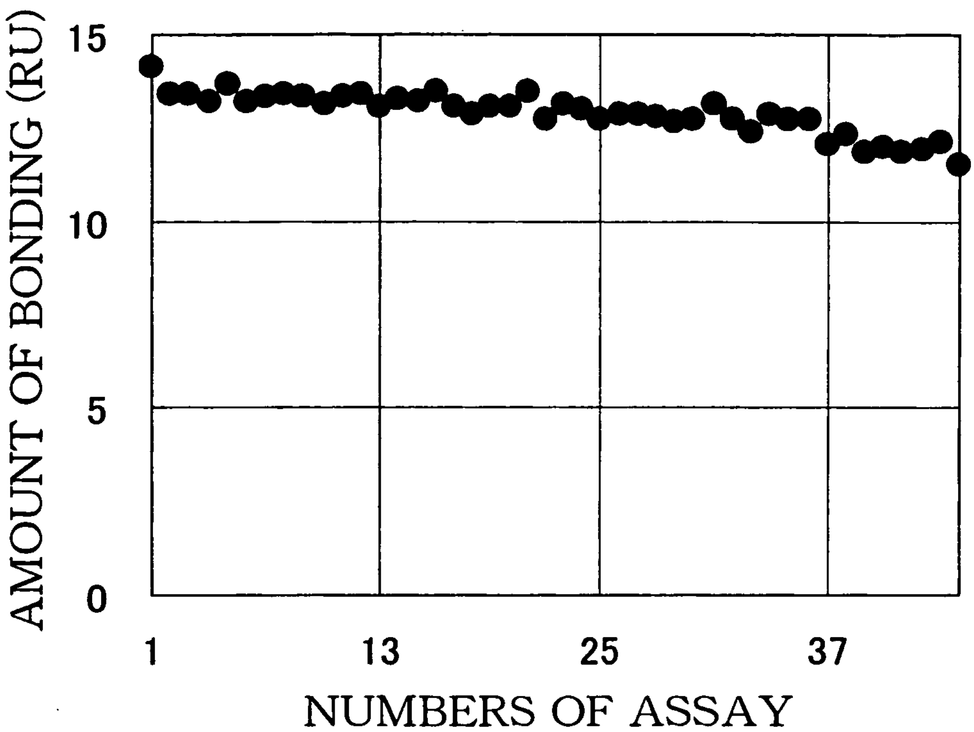

European Patent Office

# EUROPEAN SEARCH REPORT

Application Number
EP 07 01 2263

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 98/33941 A (NEXSTAR PHARMACEUTICALS INC [US]) 6 August 1998 (1998-08-06)<br>* page 6, line 6 - page 7, line 16 *<br>* page 7, line 19 - page 9, line 14 *<br>* page 13, line 30 - page 15, line 15 *<br>* page 17 *<br>* page 19 *<br>* claims 1-13 *<br>----- | 1-4 | INV.<br>G01N33/543<br>G01N33/551<br>B01J19/00 |
| X | EP 0 517 001 A (HEWLETT PACKARD CO [US] HEWLETT PACKARD CO [DE])<br>9 December 1992 (1992-12-09)<br>* page 4, line 42 - page 7, line 31 *<br>* figure 6 *<br>* page 8, lines 25-40 *<br>* page 9, line 35 - page 11, line 26 *<br>* tables I-IV *<br>----- | 1-4 | |
| X | US 4 937 200 A (KUMAZAWA TOSHIAKI [JP] ET AL) 26 June 1990 (1990-06-26)<br>* the whole document *<br>----- | 1-4 | TECHNICAL FIELDS SEARCHED (IPC) |
| X | MARKGREN P O ET AL: "Screening of compounds interacting with HIV-1 proteinase using optical biosensor technology."<br>ANALYTICAL BIOCHEMISTRY 15 DEC 1998, vol. 265, no. 2,<br>15 December 1998 (1998-12-15), pages 340-350, XP008082111<br>ISSN: 0003-2697<br>* abstract *<br>-----<br>-/-- | 1-4 | B01J<br>G01N |

The present search report has been drawn up for all claims

| Place of search | Date of completion of the search | Examiner |
|---|---|---|
| The Hague | 7 August 2007 | Boiangiu, Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

3

EPO FORM 1503 03.82 (P04C01)

European Patent Office

# EUROPEAN SEARCH REPORT

Application Number
EP 07 01 2263

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 01/79848 A (TNO [NL]; GAAG ABRAHAM V D [NL]; STIGTER EDWIN CORNELIS ALBERT [NL]; H) 25 October 2001 (2001-10-25)<br>* page 1, paragraph 4 - page 2, paragraph 1 *<br>* page 4, paragraph 1 - page 5, paragraph 3 *<br>* page 6, paragraph 4 - page 7, last paragraph *<br>* page 12, paragraph 1 - page 13, paragraph 7 *<br>* claims 10-15 *<br>----- | 1-4 | |
| Y | WO 99/06835 A (UAB RESEARCH FOUNDATION [US]; ASANOV ALEXANDER N [US]; WILSON W WILLIA) 11 February 1999 (1999-02-11)<br>* page 13, paragraph 2 - page 16, last paragraph *<br>----- | 1-4 | |
| Y | US 2004/115709 A1 (MOROZOV VICTOR [US] ET AL) 17 June 2004 (2004-06-17)<br>* claims 1-20 *<br>* the whole document *<br>----- | 1-4 | TECHNICAL FIELDS SEARCHED (IPC) |

The present search report has been drawn up for all claims

3

| Place of search | Date of completion of the search | Examiner |
|---|---|---|
| The Hague | 7 August 2007 | Boiangiu, Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

**ANNEX TO THE EUROPEAN SEARCH REPORT ON EUROPEAN PATENT APPLICATION NO.** EP 07 01 2263

This annex lists the patent family members relating to the patent documents cited in the above-mentioned European search report.
The members are as contained in the European Patent Office EDP file on
The European Patent Office is in no way liable for these particulars which are merely given for the purpose of information.

07-08-2007

| Patent document cited in search report | Publication date | Patent family member(s) | Publication date |
|---|---|---|---|
| WO 9833941 A | 06-08-1998 | AU 6261798 A | 25-08-1998 |
| | | US 5861254 A | 19-01-1999 |
| EP 0517001 A | 09-12-1992 | JP 5232113 A | 07-09-1993 |
| | | US 5229301 A | 20-07-1993 |
| US 4937200 A | 26-06-1990 | DE 3711894 A1 | 15-10-1987 |
| | | JP 2095895 C | 02-10-1996 |
| | | JP 8007217 B | 29-01-1996 |
| | | JP 62237356 A | 17-10-1987 |
| WO 0179848 A | 25-10-2001 | AU 4694301 A | 30-10-2001 |
| WO 9906835 A | 11-02-1999 | AU 3902997 A | 22-02-1999 |
| US 2004115709 A1 | 17-06-2004 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 3294605 B